# EUROPEAN PATENT APPLICATION

(11) **EP 4 620 405 A1**
(43) Date of publication of application: **24.09.2025**
(21) Application number: 25164798.8
(22) Date of filing: 19.03.2025
(51) Int. Cl.: A61B 8/08, A61B 8/00

(54) **METHOD FOR PROVIDING GUIDELINE FOR CARDIAC ULTRASOUND IMAGE AND DEVICE FOR PROVIDING GUIDELINE FOR CARDIAC ULTRASOUND IMAGE USING THE SAME**

(30) Priority: 20.03.2024 KR 20240038635; 14.03.2025 KR 20250033399; 14.03.2025 KR 20250033355
(71) Applicant: Ontact Health Co., Ltd., Seoul 03764 (KR)
(72) Inventor: LEE, Seung Ah, Seoul (KR); JEON, Jae Ik, Seoul (KR); JEONG, Hyun Seok, Seoul (KR)
(74) Representative: Müller Schupfner & Partner Patent- und Rechtsanwaltspartnerschaft mbB (Muc)

(57) **Abstract**

The present disclosure provides a method for providing a guideline for a cardiac ultrasound image implemented by a processor, in which the method includes receiving a cardiac ultrasound image of a captured subject and determining probe guidance based on the received cardiac ultrasound image by using a prediction model trained to determine probe guidance according to movement of an ultrasound probe by inputting the cardiac ultrasound image. Moreover, the present disclosure provides a device using the method.

## Description

### CROSS-REFERENCE TO RELATED APPLICATIONS

This application claims the priority of Korean Patent Applications No. 10-2025-0033355 filed on March 14, 2025, No. 10-2025-0033399 filed on March 14, 2025 and No. 10-2024-0038635 filed on March 20, 2024 in the Korean Intellectual Property Office, the disclosure of which is incorporated herein by reference.

### BACKGROUND

### Field

The present disclosure relates to a method for providing a guideline for a cardiac ultrasound image and a device for providing a guideline for a cardiac ultrasound image using the same.

### Description of the Related Art

A cardiac ultrasound examination is performed by projecting ultrasound waves on a three-dimensional structure of a heart in multiple planes to obtain images of the heart and measure hemodynamic variables.

In this case, a medical staff positions an ultrasound probe in a location where it is easy to obtain ultrasound images, so as to obtain multi-faceted images through anatomical structures around the heart, such as between the ribs, and records the images by finding the appropriate slice through rotation and tilting.

Furthermore, a measurement value associated with morphological changes in the heart can be determined from appropriate cross-sectional images, and specific diseases can be diagnosed from the measurement value. In other words, the measurement value can be provided as clinical values for diagnosing heart diseases and further for observing prognosis.

Meanwhile, the determination of the measurement value through the cardiac ultrasound examination may be dependent on the interpretation of the medical staff, and the reliability of the results may also depend on the skills of the medical staff.

That is, since the measurement values may vary greatly depending on the skill level of the medical staff, there is a continuous need for the development of a new guideline providing system that can derive highly accurate measurements from cardiac ultrasound images.

The background technology of the present disclosure has been written to facilitate understanding of the present disclosure. It should not be understood that the matters described in the background technology of the disclosure are recognized as prior art.

### SUMMARY

Meanwhile, in order to solve the above-mentioned problem, the inventors of the present disclosure have attempted to develop a guideline providing system based on an artificial neural network trained to segment a plurality of areas for a cardiac ultrasound image.

In this regard, the inventors of the present disclosure have noted that for various measurement values, a B-mode image is acquired and an M-mode or Doppler (color Doppler) image is determined therefrom, and a user performs a process for measurement within the image to determine the measurement value.

The inventors of the present disclosure have recognized that by applying an artificial neural network, a clinical process can be supplemented to provide information by selecting a frame in which the measurement value can be determined without additional work for mode switching, and highly reliable information can be provided.

Moreover, the inventors of the present disclosure have recognized that by applying an artificial neural network, it is possible to solve the problem of conventional processes having difficulty in determining measurement values due to time constraints in emergency situations.

As a result, the inventors of the present disclosure have developed a guideline providing system based on an artificial neural network.

Accordingly, the inventors of the present disclosure have expected that by providing a new guideline providing system, it is possible to provide highly reliable analysis results for the cardiac ultrasound image regardless of a skill level of the medical staff.

In particular, the inventors of the present disclosure have expected that fast and highly accurate decision-making is possible when the new guideline providing system is applied to a small device including a handheld type that has limitations in the work for acquiring the measurement value.

Accordingly, an object of the present disclosure is to provide a method for providing a guideline for a cardiac ultrasound image configured to provide information on determinable measurement parameters by segmenting the anatomical structure of the heart from a received cardiac ultrasound image using an artificial neural network-based prediction model, and a device using the same.

Objects of the present disclosure are not limited to the objects mentioned above, and other objects not mentioned will be clearly understood by those skilled in the art from the description below.

In order to solve the aforementioned problem, a method for providing a guideline for a cardiac ultrasound image according to one embodiment of the present disclosure is provided.

The method is a method for providing a guideline for a cardiac ultrasound image implemented by a processor, the method including: receiving a cardiac ultrasound image of a captured subject; and determining probe guidance based on the received cardiac ultrasound image by using a prediction model trained to determine probe guidance according to movement of an ultrasound probe by inputting the cardiac ultrasound image.

According to an aspect of the present disclosure, the method may further include receiving a cross-sectional view of the cardiac ultrasound image, in which the determining the probe guidance may include determining the probe guidance based on the received cardiac ultrasound image and the received cross-sectional view using the prediction model.

According to another aspect of the present disclosure, the determining the probe guidance may include determining first probe guidance and second probe guidance according to the movement of the probe based on the received cardiac ultrasound image using the prediction model.

According to still another aspect of the present disclosure, the second probe guidance may be defined as guidance with greater movement of the probe than the first probe guidance, the first probe guidance may include at least one probe operation guidance of Hold, Probe Head Tilt Down, Probe Head Tilt Up, Probe Head Rock Right, Probe Head Rock Left, Probe Head Tilt Right, Probe Head Tilt Left, Probe Head Rock Down, Probe Head Rock Up, Probe Rotate Clockwise, and Probe Rotate Counter-clockwise, and the second probe guidance may include at least one probe operation guidance of Slide Up, Slide Down, Slide Left, and Slide Right.

According to still another aspect of the present disclosure, the prediction model may be configured to probabilistically predict each of the first probe guidance and the second probe guidance, and the method may further include providing the first probe guidance, and selectively providing the second probe guidance when probability for the first probe guidance is equal to or greater than a predetermined level.

According to still another aspect of the present disclosure, the prediction model may be further configured to segment an anatomical structure of the heart by inputting the cardiac ultrasound image, and the determining the probe guidance may further include segmenting the anatomical structure in the received cardiac ultrasound image using the prediction model, and determining the probe guidance based on a segmentation result using the prediction model.

According to still another aspect of the present disclosure, the prediction model may be further configured to classify a cross-sectional view of the input cardiac ultrasound image by inputting the cardiac ultrasound image, and the determining the probe guidance may further include classifying a cross-sectional view of the received cardiac ultrasound image using the prediction model, and determining probe guidance corresponding to the classified cross-sectional view using the prediction model.

According to still another aspect of the present disclosure, the classifying the cross-sectional view may include classifying the received cardiac ultrasound image into at least one cross-sectional view of PLAX, PSAX-AV, PSAX MV, PSAX PM, PSAX APEX, A4C, A3C, and A2C using the prediction model, and the determining the corresponding probe guidance may include determining probe guidance for the at least one cross-sectional view.

In order to achieve the aforementioned objects, a device for providing a guideline for a cardiac ultrasound image according to another embodiment of the present disclosure is provided.

The device includes: a communication unit configured to receive a cardiac ultrasound image of a captured subject; and a processor functionally connected to the communication unit.

In this case, the processor may be configured to determine probe guidance based on the received cardiac ultrasound image using a prediction model trained to determine probe guidance according to movement of an ultrasound probe by inputting the cardiac ultrasound image.

According to an aspect of the present disclosure, the prediction model may be a model trained to determine the probe guidance by inputting the cardiac ultrasound image and a cross-sectional view of the cardiac ultrasound image.

According to still another aspect of the present disclosure, the processor may be further configured to determine first probe guidance and second probe guidance according to the movement of the probe based on the received cardiac ultrasound image using the prediction model.

According to still another aspect of the present disclosure, the prediction model may be configured to probabilistically predict each of the first probe guidance and the second probe guidance, and the processor may provide the first probe guidance and further include an output unit configured to selectively provide the second probe guidance when probability for the first probe guidance is equal to or greater than a predetermined level.

According to still another aspect of the present disclosure, the prediction model may be further configured to segment an anatomical structure of the heart by inputting a cardiac ultrasound image, and the processor may be further configured to segment the anatomical structure in the received cardiac ultrasound image using the prediction model, and determine the probe guidance based on a segmentation result using the prediction model.

According to still another aspect of the present disclosure, the prediction model may be further configured to classify a cross-sectional view of the input cardiac ultrasound image by inputting a cardiac ultrasound image, and the processor may be further configured to classify a cross-sectional view of the received cardiac ultrasound image by using the prediction model, and determine probe guidance corresponding to the classified cross-sectional view by using the prediction model.

According to still another aspect of the present disclosure, the processor may be further configured to classify the received cardiac ultrasound image into at least one cross-sectional view of PLAX, PSAX-AV, PSAX MV, PSAX PM, PSAX APEX, A4C, A3C and A2C using the prediction model, and determine probe guidance for the at least one cross-sectional view.

In order to achieve the aforementioned object, a method for providing a guideline for a cardiac ultrasound image according to still another embodiment of the present disclosure is provided.

The method is a method for providing a guideline for a cardiac ultrasound image implemented by a processor, the method including: receiving a cardiac ultrasound image of a captured subject; segmenting an anatomical structure of the heart by inputting the cardiac ultrasound image and using a prediction model trained to classify a cross-sectional view of the input cardiac ultrasound image, thereby segmenting the anatomical structure in the received cardiac ultrasound image; classifying the cross-sectional view of the received cardiac ultrasound image using the prediction model; and determining an evaluation score for the received cardiac ultrasound image based on the segmentation result and the classified cross-sectional view.

According to an aspect of the present disclosure, the determining the evaluation score for the cardiac ultrasound image may include determining a centering distance for the segmented anatomical structure, determining an uncertainty score for the segmented anatomical structure, determining an uncertainty score for the classified cross-sectional view, and determining the evaluation score based on the centering distance, the uncertainty score for the structure, and the uncertainty score for the cross-sectional view.

According to still another aspect of the present disclosure, the determining the uncertainty score for the structure may include determining a first uncertainty score for all of the segmented anatomical structures, and determining a second uncertainty score for each of the segmented anatomical structures.

According to still another aspect of the present disclosure, the determining the evaluation score for the cardiac ultrasound image may include determining a structural score defined as a score for evaluating capture of the anatomical structure for the received cardiac ultrasound image based on the segmentation result and the classified cross-sectional view, and determining an image quality score defined as a score for evaluating an image quality for the cardiac ultrasound image.

According to still another aspect of the present disclosure, the prediction model may be further configured to determine probe guidance according to movement of the ultrasound probe by inputting the cardiac ultrasound image, and may further include determining the probe guidance based on the segmentation result and the classified cross-sectional view using the prediction model.

According to still another aspect of the present disclosure, the prediction model may be a model trained to determine the probe guidance by inputting the cardiac ultrasound image and the cross-sectional view of the cardiac ultrasound image.

According to still another aspect of the present disclosure, the determining the probe guidance may include determining the first probe guidance and the second probe guidance according to the movement of the probe based on the segmentation result and the classified cross-sectional view using a prediction model.

According to still another aspect of the present disclosure, the prediction model may be a model trained to classify an odd cardiac ultrasound image by inputting cardiac ultrasound images.

According to still another aspect of the present disclosure, the cardiac ultrasound image may be a video including a plurality of frames.

In order to achieve the aforementioned objects, a device for providing a guideline for a cardiac ultrasound image according to still another embodiment of the present disclosure is provided.

The device includes a communication unit configured to receive a cardiac ultrasound image of a captured subject and a processor functionally connected to the communication unit. In this case, the processor is configured to segment an anatomical structure of the heart by inputting the cardiac ultrasound image and using a prediction model trained to classify a cross-sectional view of the input cardiac ultrasound image, thereby segmenting the anatomical structure in the received cardiac ultrasound image, classify the cross-sectional view of the received cardiac ultrasound image using the prediction model, and determine an evaluation score for the received cardiac ultrasound image based on the segmentation result and the classified cross-sectional view.

According to an aspect of the present disclosure, the processor may be further configured to determine a centering distance for the segmented anatomical structure, determine an uncertainty score for the segmented anatomical structure, determine an uncertainty score for the classified cross-sectional view, and determine the evaluation score based on the centering distance, the uncertainty score for the structure, and the uncertainty score for the cross-sectional view.

According to another aspect of the present disclosure, the processor may be further configured to determine a first uncertainty score for all of the segmented anatomical structures, and determine a second uncertainty score for each of the segmented anatomical structures.

According to still another aspect of the present disclosure, the processor may be further configured to determine the structural score for the received cardiac ultrasound image based on the segmentation result and the classified cross-sectional view, and to determine the image quality score for the cardiac ultrasound image.

According to still another aspect of the present disclosure, the prediction model may be trained to determine guidance according to movement of a probe, and the processor may be further configured to determine the probe guidance based on the segmentation result and the classified cross-sectional view using the prediction model.

According to still another aspect of the present disclosure, the processor may be further configured to determine the first probe guidance and the second probe guidance according to the movement of the probe based on the segmentation result and the classified cross-sectional view using the prediction model.

According to still another aspect of the present disclosure, the second probe guidance may be defined as guidance with greater movement of the probe than the first probe guidance, and the processor may be further configured to provide appropriate probe guidance according to the skill level of a user.

According to still another aspect of the present disclosure, the prediction model may be a model trained to classify an odd cardiac ultrasound image by inputting the cardiac ultrasound image, and the processor may be further configured to determine whether the captured cardiac ultrasound image maintains a normal cross-sectional view.

Specific details of other embodiments are included in the detailed description and drawings.

The present disclosure may provide a system for providing a guideline for a cardiac ultrasound image based on an artificial neural network, which enables acquisition of the measurement value of the heart based on the anatomical structure of the heart using the cardiac ultrasound image.

In particular, the present disclosure provides a guideline providing system that provides information on a frame in which a measurement conforming to an ASE guideline can be determined within a plurality of divided areas, thereby enabling faster and more accurate quantification results of cardiac structures.

Furthermore, the present disclosure is applicable to a small device including a handheld type that has limitations in performing a task for obtaining the measurement value, particularly a process for obtaining a specific image, and can provide fast and reliable information using the small device.

In addition, the present disclosure provides a prediction model trained to determine probe guidance according to the movement of the ultrasound probe by inputting the cardiac ultrasound image, thereby improving the quality of the cardiac ultrasound image and implementing a guideline providing method that can guide optimal probe operation tailored to the skill level of the user.

According to the present disclosure, probe operation may be analyzed in real time based on the captured cardiac ultrasound image, and optimal guidance may be determined through the prediction model. Through this, the present disclosure may support an examiner to obtain more accurate ultrasound images, and in particular, may provide customized probe operation guidance suitable for both a beginner and an expert.

In addition, the present disclosure may provide a more quantitative and objective photographing assistance system than the existing experience-based ultrasound photographing method by automatically analyzing the movement of the ultrasound probe through the prediction model and determining probe guidance considering the image quality and alignment of anatomical structures.

Furthermore, the guideline providing method of the present disclosure provides feedback in real time so that the examiner may immediately adjust the probe operation, thereby reducing unnecessary shooting errors during the examination process and providing the effect of shortening the ultrasound examination time.

As a result, the present disclosure may contribute to improving the accuracy of diagnosis by improving the accuracy and reproducibility of cardiac ultrasound examinations and helping medical staff obtain more stable and reliable cardiac ultrasound images. In other words, the present disclosure may provide highly reliable analysis results for cardiac ultrasound images regardless of the skill level of the examiner, and may contribute to establishing more accurate decision-making and treatment plans at the image analysis stage.

In addition, the present disclosure provides the prediction model that automatically segments anatomical structures of the heart and classifies cross-sectional views, thereby enabling rapid and accurate recognition of specific anatomical structures in ultrasound images. This enables acquisition of more standardized cardiac ultrasound images without relying on the experience of the examiner.

Furthermore, the present disclosure automatically calculates an evaluation score based on the segmentation result and cross-sectional view classification of the image, thereby enabling quantitative evaluation of the quality of a captured ultrasound image in real time.

Effects according to the present disclosure are not limited to those exemplified above, and more diverse effects are included in the present specification.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIGS. 1A and 1B illustrate a system for providing a guideline for a cardiac ultrasound image using a device for providing a guideline for the cardiac ultrasound image according to one embodiment of the present disclosure.
FIG. 2A is a block diagram illustrating the configuration of a medical staff device according to one embodiment of the present disclosure.
FIG. 2B is a block diagram illustrating the configuration of a server for providing a guideline according to one embodiment of the present disclosure.
FIG. 3 illustrates a procedure of a method for providing a guideline for a cardiac ultrasound image according to one embodiment of the present disclosure.
FIGS. 4A and 4C and FIGS. 5 to 7 exemplarily illustrate a procedure of the method for providing the guideline for the cardiac ultrasound image according to one embodiment of the present disclosure.
FIG. 8 exemplarily illustrates a user interface based on a method for providing a guideline according to various embodiments of the present disclosure.
FIGS. 9A and 9B and FIGS. 10A to 10C illustrate an evaluation result of a prediction model used in the method for providing the guideline according to various embodiments of the present disclosure.
FIGS. 11A to 11F exemplarily illustrate a procedure of a method for providing a guideline for a cardiac ultrasound image according to another embodiment of the present disclosure.
FIGS. 12A to 12C exemplarily illustrate a user interface based on the method for providing the guideline according to various embodiments of the present disclosure.
FIGS. 13A to 13E exemplarily illustrate a procedure of a method for providing a guideline for a cardiac ultrasound image according to another embodiment of the present disclosure.
FIGS. 14A to 14D illustrate an evaluation result of a prediction model capable of performing anatomical structure segmentation and cross-sectional view classification used in a method for providing a guideline according to various embodiments of the present disclosure.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

The advantages of the disclosure and the method for achieving them will become apparent by referring to the embodiments described in detail below together with the accompanying drawings. However, the present disclosure is not limited to the embodiments disclosed below, but may be implemented in various different forms, and these embodiments are provided only to make the disclosure of the present disclosure complete and to fully inform those skilled in the art of the scope of the disclosure.

The shapes, sizes, ratios, angles, numbers, or the like disclosed in the drawings for explaining embodiments of the present disclosure are exemplary, and therefore the present disclosure is not limited to the matters illustrated. In addition, when describing the present disclosure, when it is determined that a detailed description of a related known technology may unnecessarily obscure the gist of the present disclosure, the detailed description will be omitted. When the terms "include", "have", "consist of", and the like are used in this specification, other parts may be added unless "only" is used. When a component is expressed in the singular, it includes a case where the plural is included unless there is a specifically explicit description.

When interpreting components, it is interpreted as including the error range even when there is no separate explicit description.

The individual features of the various embodiments of the present disclosure may be partially or wholly combined or combined with each other, and as can be fully understood by those skilled in the art, various technical connections and operations are possible, and each embodiment may be implemented independently of each other or may be implemented together in a related relationship.

For clarity in the interpretation of this specification, the terms used in this specification are defined below.

The term "subject" as used in the present specification may mean any subject that seeks to receive guideline information for determining the measurement value from the cardiac ultrasound image. Meanwhile, the subject disclosed in this specification may be any mammal other than a human, but is not limited thereto.

As used herein, the term "cardiac ultrasound image" refers to a cardiac ultrasound image of the subject, which may be a still cut image of a single frame or a moving image composed of multiple frames.

In this case, the cardiac ultrasound image may be a 2D or 3D image.

According to an aspect of the present disclosure, the cardiac ultrasound image may be a B-mode cardiac ultrasound image that is the basis for determining the measurement value. However, it is not limited thereto, and the cardiac ultrasound image may include a Doppler cardiac ultrasound image, a color Doppler cardiac ultrasound image, or an M-mode cardiac ultrasound image that enables determination of the measurement value.

As used herein, the term "anatomical structure" may refer to an anatomical structure of the heart that can be identified in a cardiac ultrasound image.

According to an aspect of the present disclosure, the anatomical structure may include an area selected from the following: a right ventricle anterior wall, a right ventricle (RV), an anterior wall of aorta, an aorta, a posterior wall of aorta, a left atrium (LA), and a posterior wall of a left atrium (LA). However, the present disclosure is not limited thereto.

Here, a "parameter" or "measurement value" may mean a value, such as a measurable thickness, diameter, or the like, of a cardiac structure, and may be used interchangeably herein.

According to an aspect of the present disclosure, the parameter may be at least one of an interventricular septum thickness (IVS), a left ventricular diameter (LVID), a left ventricular posterior wall diastole (LVPWD), a right ventricular outflow tract (RVOT), an aortic diameter (Ao), and a left atrium diameter (LA). However, the present disclosure is not limited thereto.

In this case, a "measurable parameter" may be defined as a measurement parameter for which a determination of a value corresponding to the parameter is possible.

As used herein, the term "mode parameter" may be defined as a parameter measurable from a mode selected by the user.

According to various embodiments of the present disclosure, a prediction model trained to segment anatomical structures for frames in a cardiac ultrasound image is presented.

As used herein, the term "prediction model" may be a model configured to segment the anatomical structure and output the segmented anatomical structure by inputting the cardiac ultrasound image.

Meanwhile, the output value of the prediction model is not limited to those described above.

For example, the prediction model may be a model trained to output the location (coordinates) of each anatomical structure by inputting the cardiac ultrasound image.

As another example, the prediction model may be a model trained to segment the area, and output a probability for each anatomical structure corresponding to each area by inputting the cardiac ultrasound image.

According to an aspect of the present disclosure, the prediction model may be a model further trained to determine and output a location-based connectivity meter, an uncertainty score corresponding to uncertainty, and further a capacity meter corresponding to a volume for each of the segmented anatomical structures.

Here, the uncertainty score may include entropy, mutual information between predictions and model posterior (for example, Bayesian Active Learning by Disagreement (BALD), variation ratios, and mean standard deviation), which indicates how much the probability variables of the "prediction" and "model posterior probability" are related to each other.

Additionally, the prediction model may include a model trained to classify the type of cross-section, and output results and probabilities by inputting the cardiac ultrasound image.

According to another aspect of the present disclosure, scores for each of the connectivity meter, the uncertainty, and the capacity meter may be calculated, and quality evaluation of an image may be performed based on a combination of these scores.

In various embodiments of the present disclosure, measurable parameters may be obtained from the corresponding image based on the positional relationship of the anatomical structures within the cardiac ultrasound image.

According to another aspect of the present disclosure, a measurable image mode may be obtained from the corresponding image based on the positional relationship of each anatomical structure.

According to another aspect of the present disclosure, a guideline for obtaining a measurable image mode from a corresponding image may be determined based on the positional relationship of each anatomical structure. For example, a line for obtaining a Doppler mode or color Doppler mode image, or a box for obtaining an M-mode image may be determined and displayed within a basic image. Accordingly, a user may more easily obtain images of other modes for determining measurement values. That is, images of various modes may be obtained and the measurement values may be determined using a handheld type device with a smaller screen than a general ultrasound diagnostic device.

In this case, determination of measurable parameters or determination of measurable modes may be performed by a prediction model.

That is, the prediction model may be a model further trained to segment anatomical structures in the input cardiac ultrasound image and, at the same time, predict measurable parameters or measurable modes from the image based on the segmentation results.

However, this is not limited to the above, and prediction of measurable parameters or measurable modes from images may be possible by a measurement value prediction model that exists independently of the prediction model and is trained to predict measurable parameters or modes by taking as input the segmentation results of the prediction model.

For example, the measurement value prediction model may probabilistically predict the measurable parameter or measurable mode.

According to an aspect of the present disclosure, when a selection for the measurable ultrasound mode is input from a user, an image corresponding to the selected mode and values for measurable parameters within the image may be output and provided to the user.

Furthermore, the prediction model includes an output layer consisting of a plurality of nodes, where the number of plurality of nodes may correspond to the number of areas (number of classes) of predetermined anatomical structures.

According to another aspect of the present disclosure, the prediction model is based on deep learning-based learning, and based on the results thereof, measurable parameters, measurable other modes (M-mode, Doppler, color Doppler), or the like may be determined based on a rule base, but the rule base stage is not limited thereto and may be based on machine learning-based learning.

In more various embodiments of the present disclosure, the prediction model may be a model trained to determine the probe guidance according to movement of the ultrasound probe by inputting the cardiac ultrasound image.

In more various embodiments of the present disclosure, the prediction model may be a model trained to determine probe guidance according to ultrasound probe movement by inputting the cardiac ultrasound image and the cross-sectional view of the image.

In this case, the prediction model may be a model trained to determine the probe guidance by inputting the cardiac ultrasound image and the cross-sectional view of the cardiac ultrasound image.

In various embodiments of the present disclosure, the prediction model may be a model trained to probabilistically predict probe guidance.

Here, the probe guidance (probe manipulation guide) in ultrasound examination is a system that guides how to adjust the probe to obtain the correct image, and may include first probe guidance for a beginner and second probe guidance for advanced depending on movement of a probe.

More specifically, the first probe guidance may be different for each mode.

In various embodiments of the present disclosure, the first probe guidance may include at least one probe operation guidance of Hold, Probe Head Tilt Down, Probe Head Tilt Up, Probe Head Rock Right, Probe Head Rock Left, Probe Rotate Clockwise, and Probe Rotate Counter-clockwise in PLAX and A4C shooting modes.

In various embodiments of the present disclosure, the first probe guidance may include at least one probe operation guidance of Hold, Probe Head Tilt Right, Probe Head Tilt Left, Probe Head Rock Down, Probe Head Rock Up, Probe Rotate Clockwise, and Probe Rotate Counter-clockwise in PSAX, A2C, A3C, and IVC shooting modes.

In various embodiments of the present disclosure, the second probe guidance may provide a more sophisticated probe operation method and include at least one probe operation guidance of Slide Up, Slide Down, Slide Left, and Slide Right to provide precise scanning of anatomical structures.

For example, a probe operation guide based on the first guidance may be received so as to learn basic operations such as Tilt, Rock, and Rotation and find a standard view in a state of fixing a starting point position of the probe. Moreover, when fine correction is still needed even though a standard view is formed, a probe operation guide based on the second guidance may be received so as to obtain an optimal ultrasound image by performing a sliding operation through greater movement.

However, it is not limited thereto, and the probe guidance may include more various probe operation guidance according to the cross-sectional view.

Meanwhile, the prediction model may be, but is not limited to, a model configured to determine the probe guidance based on segmentation of the anatomical structure and/or classification of cross-sectional view.

In more various embodiments of the present disclosure, the prediction model may be a model trained to classify an odd cardiac ultrasound image by inputting the cardiac ultrasound image.

That is, the prediction model may be used to maintain the correct cross-sectional view and detect abnormalities in the captured cardiac ultrasound image. Meanwhile, the prediction model may be based on at least one algorithm selected from among DenseNet-121, U-net, VGG net, DenseNet, Fully Convolutional Network (FCN) with encoder-decoder structure, deep neural network (DNN) such as SegNet, DeconvNet, or DeepLAB V3+, Transformer such as Lawin+, SegFormer, or Swin, SqueezeNet, Alexnet, ResNet18, MobileNet-v2, GoogLeNet, Resnet-v2, Resnet50, RetinaNet, Resnet101, Inception-v3, HRNet, ResNeXt, EfficientNet. Furthermore, the prediction model may be an ensemble model based on at least two algorithm models of the above-mentioned algorithms. However, it is not limited thereto.

In more various embodiments of the present disclosure, the cardiac ultrasound image may be a video including a plurality of frames, and the prediction model may be a fusion model of a temporal model that trains changes in time or between consecutive frames and a 2D model that analyzes a single image or independent frames.

For example, when a 2D CNN analyzes an individual frame of the cardiac ultrasound image to extract immediate structural information, the temporal model may perform a comprehensive analysis of the cardiac ultrasound image by linking multiple frames to analyze changes over time.

Accordingly, it is possible to provide optimal ultrasound probe guidance that takes into account not only the quality evaluation of a single image, but also the continuous flow of images.

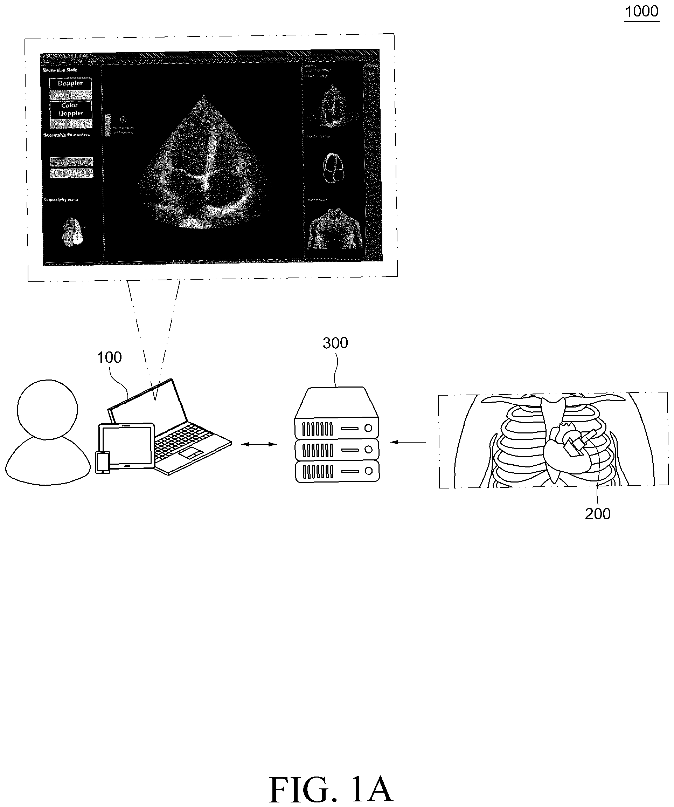

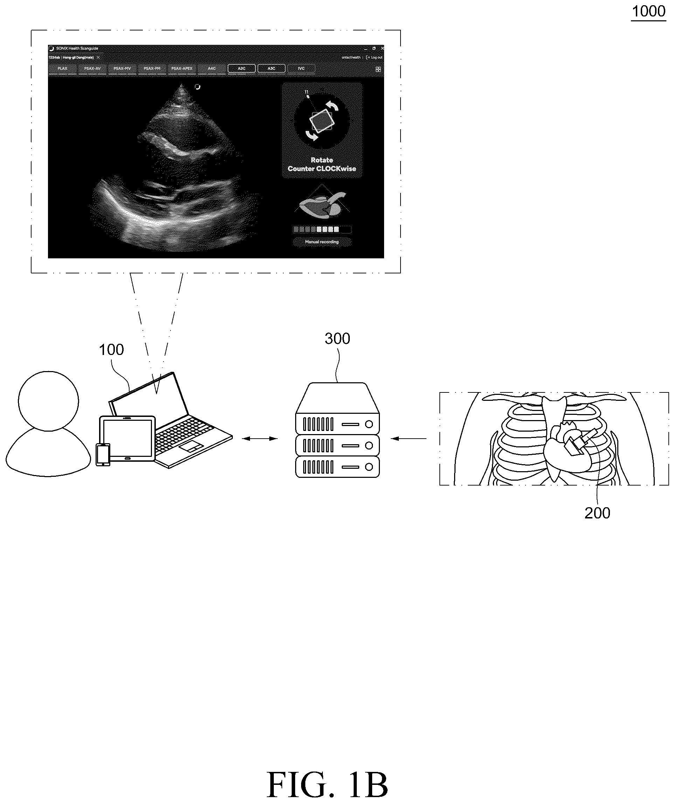

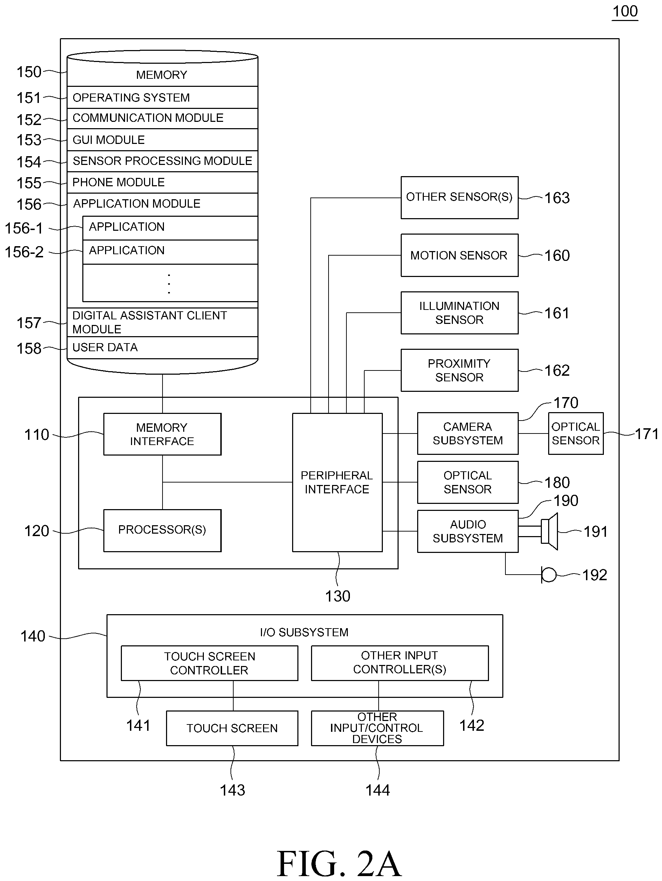

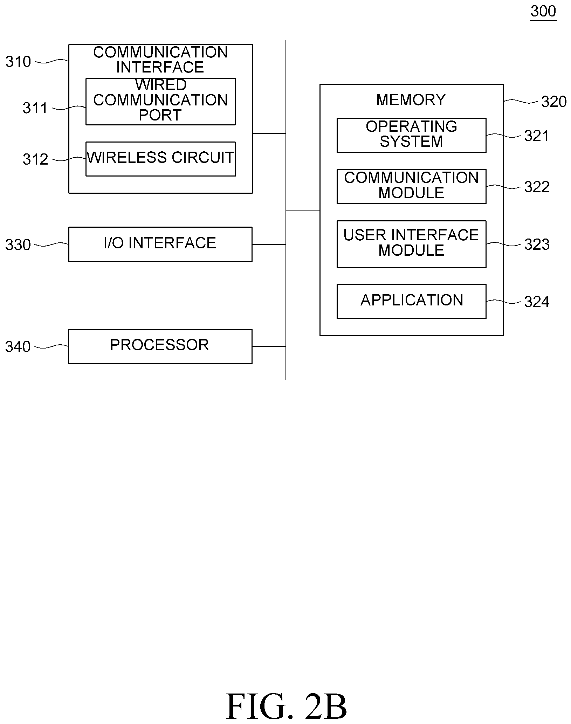

Hereinafter, with reference to FIGS. 1A, 1B, 2A and 2B, the system for providing the guideline for the cardiac ultrasound image and the device for providing the guideline for the cardiac ultrasound image using the device for providing the guideline for the cardiac ultrasound image according to one embodiment of the present disclosure will be described.

FIGS. 1A and 1B illustrate the system for providing the guideline for the cardiac ultrasound image using the device for providing the guideline for the cardiac ultrasound image according to one embodiment of the present disclosure. FIG. 2A exemplarily illustrates a configuration of a medical staff device for receiving the guideline for the cardiac ultrasound image according to one embodiment of the present disclosure. FIG. 2B exemplarily illustrates a configuration of a server for providing the guideline for the cardiac ultrasound image according to one embodiment of the present disclosure.

First, referring to FIG. 1A, a guideline providing system 1000 may be a system configured to provide information associated with a measurement parameter based on a cardiac ultrasound image of a subject. In this case, the guideline providing system 1000 may be configured with a medical staff device 100 that receives information associated with the measurement parameter, an ultrasound image diagnosis device 200 that provides the cardiac ultrasound image, and a guideline providing server 300 that generates information associated with the measurement parameter based on the received cardiac ultrasound image.

In various embodiments of the present disclosure, the guideline providing server 300 may be mounted on the ultrasonic image diagnosis device 200, and in this case, various pieces of information related to the measurement value may be displayed on a display unit (not illustrated) of the ultrasonic image diagnosis device 200.

That is, the user may check information related to the cardiac measurement value simultaneously with the diagnosis by using the ultrasound image diagnosis device 200.

In various embodiments, the medical staff device 100 is an electronic device that provides a user interface for presenting information associated with the measurement parameter, and may include at least one of a smart phone, a tablet PC (Personal Computer), a laptop, and/or a PC.

The medical staff device 100 may receive the guideline for determining the measurement parameter for the subject from the guideline providing server 300 and display the received results through a display unit (not illustrated).

The guideline providing server 300 may include a general-purpose computer, laptop, and/or data server that performs various operations for determining information related to cardiac quantification based on a cardiac ultrasound image provided from the ultrasound image diagnosis device 200, such as an ultrasound diagnostic device. **In** this case, the guideline providing server 300 may be a device for accessing a web server that provides a web page or a mobile web server that provides a mobile website, but is not limited thereto.

More specifically, the guideline providing server 300 receives the cardiac ultrasound image from the ultrasound image diagnosis device 200 and performs prediction on the anatomical structure for determining the measurement value within the received cardiac ultrasound image. In this case, the guideline providing server 300 may perform prediction on the location of the anatomical structure within the cardiac ultrasound image using the prediction model.

Furthermore, the guideline providing server 300 may determine the measurable parameter and/or measurable mode based on the anatomical structure.

The guideline providing server 300 may provide the measurable parameter and/or measurable mode, and further segmentation result for the anatomical structure, to the medical staff device 100.

Referring to FIG. 1B together, in more various embodiments, the medical staff device 100 may receive guidelines for probe operation from the guideline providing server 300 and display the received results through a display unit (not illustrated).

More specifically, the guideline providing server 300 receives the cardiac ultrasound image and the cross-sectional view from the ultrasound image diagnosis device 200, and performs prediction of the probe guidance based on the received cardiac ultrasound image. In this case, the guideline providing server 300 may perform prediction of the probe guidance using the prediction model.

Furthermore, the guideline providing server 300 may determine probe guidance specific to a specific cross-sectional view.

In more various embodiments of the present disclosure, the guideline providing server 300 receives the cardiac ultrasound image from the ultrasound image diagnosis device 200, and performs prediction of the anatomical structure and classification of the cross-sectional view based on the received cardiac ultrasound image. In this case, the guideline providing server 300 may perform the segmentation of the anatomical structure and the classification of cross-sectional view in the cardiac ultrasound image using the prediction model.

Furthermore, the guideline providing server 300 may determine an evaluation score for the cardiac ultrasound image based on the segmented anatomical structure and cross-sectional view.

In this way, information provided from the guideline providing server 300 may be provided as a web page through a web browser installed on the medical staff device 100, or may be provided in the form of an application or program. In various embodiments, such data may be provided in a form included in a platform in a client-server environment.

Next, with reference to FIGS. 2A and 2B, the components of the guideline providing server 300 of the present disclosure will be specifically described.

First, referring to FIG. 2A, the medical staff device 100 may include a memory interface 110, one or more processors 120, and a peripheral interface 130. Various components within the medical staff device 100 may be connected by one or more communication buses or signal lines.

The memory interface 110 is connected to the memory 150 and may transmit various data to the processor 120. Here, the memory 150 may include at least one type of storage medium of flash memory type, hard disk type, multimedia card micro type, card type memory (for example, SD or XD memory, or the like), RAM, SRAM, ROM, EEPROM, PROM, network storage, cloud, and blockchain data.

In various embodiments, the memory 150 may store at least one of an operating system 151, a communication module 152, a graphical user interface module (GUI) 153, a sensor processing module 154, a telephone module 155, and an application module 156. Specifically, the operating system 151 can include instructions for processing basic system services and instructions for performing hardware operations. The communication module 152 can communicate with at least one of one or more other devices, computers, and servers. The graphical user interface module (GUI) 153 may process a graphical user interface. The sensor processing module 154 can process sensor-related functions (for example, processing voice input received using one or more microphones 192). The telephone module 155 may process telephone-related functions. The application module 156 may perform various functions of the user application, such as electronic messaging, web browsing, media processing, navigation, imaging, and other processing functions. In addition, the medical staff device 100 may store one or more software applications 156-1 and 156-2 (for example, guideline providing applications) associated with a type of service in the memory 150.

In various embodiments, the memory 150 may store a digital assistant client module 157 (hereinafter, DA client module), and thus store instructions for performing client-side functions of the digital assistant and various user data 158.

Meanwhile, the DA client module 157 may obtain the user's voice input, text input, touch input, and/or gesture input through various user interfaces (for example, I/O subsystem 140) provided in the medical staff device 100.

Additionally, the DA client module 157 may output data in audiovisual and tactile forms. For example, the DA client module 157 may output data consisting of a combination of at least two or more of voice, sound, notification, text message, menu, graphic, video, animation, and vibration. Additionally, the DA client module 157 may communicate with a digital assistant server (not illustrated) using a communication subsystem 180.

In various embodiments, the DA client module 157 may collect additional information about the surrounding environment of the medical staff device 100 from various sensors, subsystems, and peripheral devices to construct a context associated with the user input. For example, the DA client module 157 may provide context information along with the user input to a digital assistant server to infer the user's intent. Here, the context information that may accompany the user input may include sensor information, such as lighting, ambient noise, ambient temperature, images of the surrounding environment, video, or the like. As another example, the context information may include the physical state (for example, device orientation, device position, device temperature, power level, speed, acceleration, motion pattern, cellular signal strength, or the like) of the medical staff device 100. As another example, the context information may include information (for example, processes running on the medical staff device 100, installed programs, past and current network activity, background services, error logs, resource usage, or the like) related to the software state of the medical staff device 100.

In various embodiments, the memory 150 may include additional or deleted instructions, and may further include additional configurations other than those illustrated in FIG. 2A of the medical staff device 100, or may exclude some configurations.

The processor 120 may control the overall operation of the medical staff device 100 and execute various commands to implement an interface that provides information associated with measurement parameters by running an application or program stored in the memory 150.

The processor 120 may correspond to a computing device such as a central processing unit (CPU) or an application processor (AP). In addition, the processor 120 may be implemented in the form of an integrated chip IC such as a system on chip (SoC) in which various computing devices such as a neural processing unit (NPU) are integrated.

The peripheral interface 130 may be connected to various sensors, subsystems, and peripheral devices to provide data so that the medical staff device 100 can perform various functions. Here, it can be understood that the function performed by the medical staff device 100 is performed by the processor 120.

The peripheral interface 130 may receive data from a motion sensor 160, a light sensor (optical sensor) 161, and a proximity sensor 162, through which the medical staff device 100 may perform orientation, light, and proximity detection functions, or the like. For another example, the peripheral interface 130 may receive data from other sensors 163 (positioning system-GPS receiver, temperature sensor, biometric sensor), through which the medical staff device 100 may perform functions related to the other sensors 163.

In various embodiments, the medical staff device 100 may include a camera subsystem 170 connected to a peripheral interface 130 and an optical sensor 171 connected thereto, through which the medical staff device 100 may perform various shooting functions, such as taking pictures and recording video clips.

In various embodiments, the medical staff device 100 may include a communication subsystem 180 coupled with the peripheral interface 130. The communication subsystem 180 may be comprised of one or more wired/wireless networks and may include various communication ports, radio frequency transceivers, and optical transceivers.

In various embodiments, the medical staff device 100 includes an audio subsystem 190 coupled to the peripheral interface 130, the audio subsystem 190 including one or more speakers 191 and one or more microphones 192, such that the medical staff device 100 can perform voice-activated functions, such as voice recognition, voice replication, digital recording, and telephony functions.

In various embodiments, the medical staff device 100 may include an I/O subsystem 140 coupled with the peripheral interface 130. For example, the I/O subsystem 140 may control a touch screen 143 included in the medical staff device 100 via a touch screen controller 141. As an example, the touch screen controller 141 may detect a user's contact and movement or cessation of contact and movement using any one of a plurality of touch sensing technologies, such as capacitive, resistive, infrared, surface acoustic wave technology, proximity sensor array, and the like. As another example, the I/O subsystem 140 may control other input/control devices 144 included in the medical staff device 100 via other input controller(s) 142. As an example, the other input controller(s) 142 may control one or more buttons, rocker switches, thumb-wheels, infrared ports, USB ports, and pointer devices such as a stylus.

Next, referring to FIG. 2B, the guideline providing server 300 may include a communication interface 310, a memory 320, an I/O interface 330, and a processor 340, each of which may communicate with each other through one or more communication buses or signal lines.

The communication interface 310 may be connected to the medical staff device 100 and the ultrasound image diagnosis device 200 via a wired/wireless communication network to exchange data. For example, the communication interface 310 may receive the cardiac ultrasound image from the ultrasound image diagnosis device 200, determine the measurement parameters or guidelines for obtaining the same therefrom, and transmit the measurement parameters or the guidelines to the medical staff device 100.

Meanwhile, the communication interface 310 that enables transmission and reception of such data includes a communication port 311 and a wireless circuit 312, in which the wired communication port 311 may include one or more wired interfaces, for example, Ethernet, Universal Serial Bus USB, FireWire, or the like. In addition, the wireless circuit 312 may transmit and receive data with an external device via an RF signal or an optical signal. In addition, the wireless communication may use at least one of a plurality of communication standards, protocols, and technologies, for example, GSM, EDGE, CDMA, TDMA, Bluetooth, Wi-Fi, VoIP, Wi-MAX, or any other suitable communication protocol.

The memory 320 may store various data used in the guideline providing server 300. For example, the memory 320 may store the cardiac ultrasound image or the prediction model trained to segment the anatomical structure within the cardiac ultrasound image.

In various embodiments, the memory 320 may include a volatile or nonvolatile storage medium capable of storing various data, commands, and information. For example, the memory 320 may include at least one type of storage medium among a flash memory type memory, a hard disk type memory, a multimedia card micro type memory, a card type memory (for example, an SD or XD memory, or the like), a RAM, an SRAM, a ROM, an EEPROM, a PROM, a network storage, a cloud, and blockchain data.

In various embodiments, the memory 320 may store a configuration of at least one of an operating system 321, a communication module 322, a user interface module 323, and one or more applications 324.

The operating system 321 (for example, an embedded operating system such as LINUX, UNIX, MAC OS, WINDOWS, VxWorks, or the like) may include various software components and drivers to control and manage general system operations (for example, memory management, storage device control, power management, or the like) and may support communication between various hardware, firmware, and software components.

The communication module 322 may support communication with other devices through the communication interface 310. The communication module 322 may include various software components for processing data received by the wired communication port 311 or wireless circuit 312 of the communication interface 310.

The user interface module 323 may receive a user's request or input from a keyboard, touch screen, microphone, or the like through the I/O interface 330 and provide the user interface on the display.

The application 324 may include a program or module configured to be executed by one or more processors 340. Here, the application for providing information associated with the measurement parameter may be implemented on a server farm.

The I/O interface 330 may connect an input/output device (not illustrated) of the guideline providing server 300, for example, at least one of a display, a keyboard, a touch screen, and a microphone, to the user interface module 323. The I/O interface 330 may receive user input (for example, voice input, keyboard input, touch input, or the like) together with the user interface module 323 and process a command according to the received input.

The processor 340 is connected to the communication interface 310, the memory 320, and the I/O interface 330 to control the overall operation of the guideline providing server 300, and may perform various commands for providing the guideline through an application or program stored in the memory 320.

The processor 340 may correspond to a computing device such as a central processing unit (CPU) or an application processor (AP). In addition, the processor 340 may be implemented in the form of an integrated chip (IC) such as a system on chip (SoC) in which various computing devices are integrated. Alternatively, the processor 340 may include a module for calculating an artificial neural network model such as a neural processing unit (NPU).

In various embodiments, the processor 340 may be configured to predict the anatomical structure within the cardiac ultrasound image using the prediction models and automatically determine and provide the measurement value. Optionally, the processor 340 may be configured to provide information about the measurement value that can be acquired from the cardiac ultrasound image and information about the measurable mode.

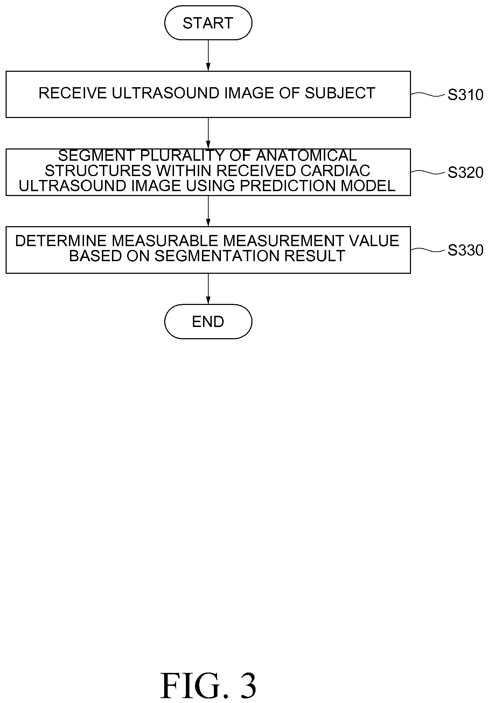

Hereinafter, with reference to FIG. 3, 4A to 4C, and 5 to 7, a method for providing the guideline for the cardiac ultrasound image according to one embodiment of the present disclosure will be specifically described.

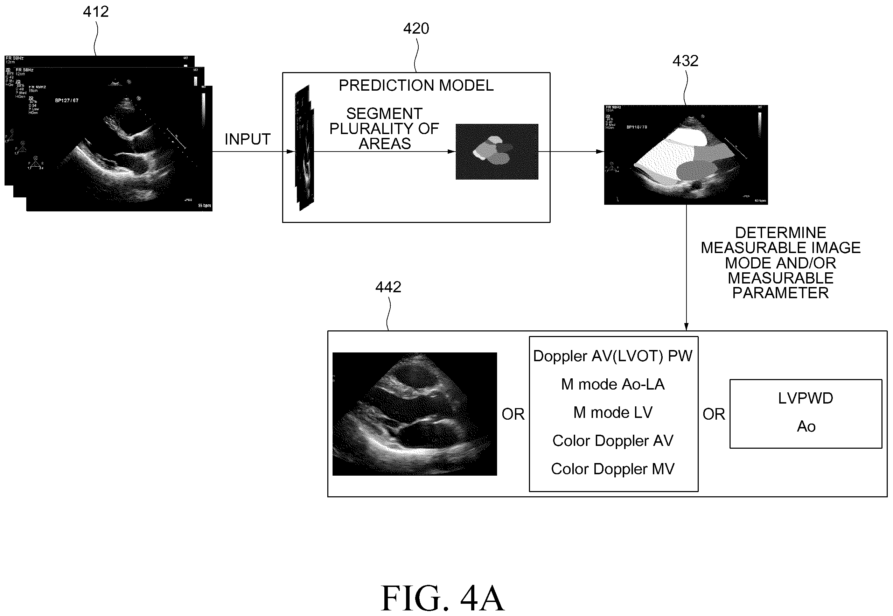

FIG. 3 illustrates a procedure of a method for providing the guideline for the cardiac ultrasound image according to one embodiment of the present disclosure. FIGS. 4A to 4C and FIGS. 5 to 7 exemplarily illustrate a procedure of the method for providing the guideline for the cardiac ultrasound image according to one embodiment of the present disclosure.

First, referring to FIG. 3, the procedure for providing the guideline according to one embodiment of the present disclosure is as follows. First, the cardiac ultrasound image of a subject is received (S310). Then, the location of the anatomical structure is determined by the prediction model (S320). Then, the measurable parameter is determined based on the anatomical structure (S330).

More specifically, in Step S310 in which the cardiac ultrasound image is received, the cardiac ultrasound image of a target site, that is, a heart site, may be received.

According to an aspect of the present disclosure, in Step S310 of receiving the cardiac ultrasound image, a cardiac ultrasound image of a still cut in B-mode or a cardiac ultrasound moving image including a plurality of frames may be received.

However, it is not limited thereto, and more various cardiac ultrasound images capable of determining the measurement value may be received at Step S310 of receiving the cardiac ultrasound image.

Next, Step S320 is performed in which the anatomical structure is predicted.

According to an aspect of the present disclosure, Step S320 of predicting the anatomical structure may be performed by a prediction model trained to segment a plurality of areas based on the anatomical structure in the cardiac ultrasound image by inputting the received cardiac ultrasound image.

That is, at Step S320 in which the anatomical structure is predicted, the location (coordinates) of the anatomical structure within the image may be determined.

In this case, the prediction model may segment at least one area selected from the right ventricle anterior wall, right ventricle (RV), anterior wall of aorta, aorta, posterior wall of aorta, left atrium (LA), and posterior wall of LA in the cardiac ultrasound image.

Next, the measurable parameter is determined based on the area-based predicted anatomical structure (S330).

According to an aspect of the present disclosure, in Step S330 in which the measurable parameter is determined, the measurable parameter may be determined based on the positional relationship of each anatomical structure.

According to another aspect of the present disclosure, in Step S330 in which the measurable parameter is determined, the measurable parameter may be determined from at least one mode of an M-mode cardiac ultrasound image, a Doppler cardiac ultrasound image, and a color Doppler cardiac ultrasound image based on a B-mode cardiac ultrasound image.

According to still another aspect of the present disclosure, a step of determining the measurable ultrasonic mode based on the segmentation result may be further performed.

In this case, in the step of determining the measurable ultrasound mode, an image quality evaluation is performed based on the segmentation result for each anatomical structure, and the measurable ultrasound mode may be determined based on the image quality evaluation.

In this case, the evaluation is performed on the location-based connectivity meter, the uncertainty score (for example, entropy meter), and further, the capacity meter corresponding to the volume for each of the segmented anatomical structures, respectively, and the score is calculated, respectively. Then, the image quality evaluation may be performed based on the total sum of each score. For example, when the sum of the scores is equal to or less than a predetermined level, it is determined as "low image quality", and when the sum is equal to or more than a predetermined level, it is determined as "good image quality" for the ultrasound mode, and the evaluation result for the image quality may be provided.

Meanwhile, in various embodiments, the connectivity meter and the capacity meter may be scored based on a rule base, and in the case of entropy, residual-based score calculation may be performed. However, this is not limited thereto.

Meanwhile, the determination of the measurable parameter, and further, the measurable mode, may be performed by the prediction model configured to segment and output the anatomical structure by inputting the cardiac ultrasound image.

That is, the prediction model may be a model further trained to segment the anatomical structure in the input cardiac ultrasound image and, at the same time, predict the measurable parameter or measurable mode from the image based on the segmentation result.

However, this is not limited to the above, and prediction of the measurable parameter or measurable mode from images may be possible by the measurement value prediction model that exists independently of the prediction model and is trained to predict the measurable parameter or modes by inputting the segmentation result of the prediction model.

For example, referring to FIG. 4A together, when a cardiac ultrasound image 412 is received, the cardiac ultrasound image is input into a prediction model 420 in Step S320 in which the anatomical structure is predicted. More specifically, a plurality of areas of the input cardiac ultrasound image 412 is segmented or features related to the anatomical structure are extracted. After an operation is performed on the extracted features, the location of the anatomical structure may be finally determined within the cardiac ultrasound image 412. Then, in Step S330 in which the measurement parameter is determined, measurement value-related information 442 including the measurable parameter and/or the measurable mode is determined based on the location of the anatomical structure in an output segmentation result 432. Here, the measurement value-related information 442 may include the anatomical structure within the image according to the segmentation, modes measurable from the image (for example, Doppler AV (LVOT) PW, M mode Ao-LA, M mode and LV, Color Doppler AV and Color Doppler MV), and measurement parameters measurable from the image (for example, LVPWD and Ao). However, the present disclosure is not limited thereto, and the measurement-related information 442 may include values for the measurable parameters, and locations of the probe (not illustrated).

That is, by utilizing the prediction model, it is possible to acquire the image in which the ultrasound image mode has been switched and secure the measurement value obtainable in more various modes without a process of determining the measurement value obtainable in the specific switched imaging mode.

Accordingly, fast and highly accurate decision making can be possible by using a small and handheld device that has limitations in the work to obtain the measurement value.

In various embodiments, the prediction model may be a model trained to segment areas for the anatomical structure and simultaneously predict information related to the measurement value.

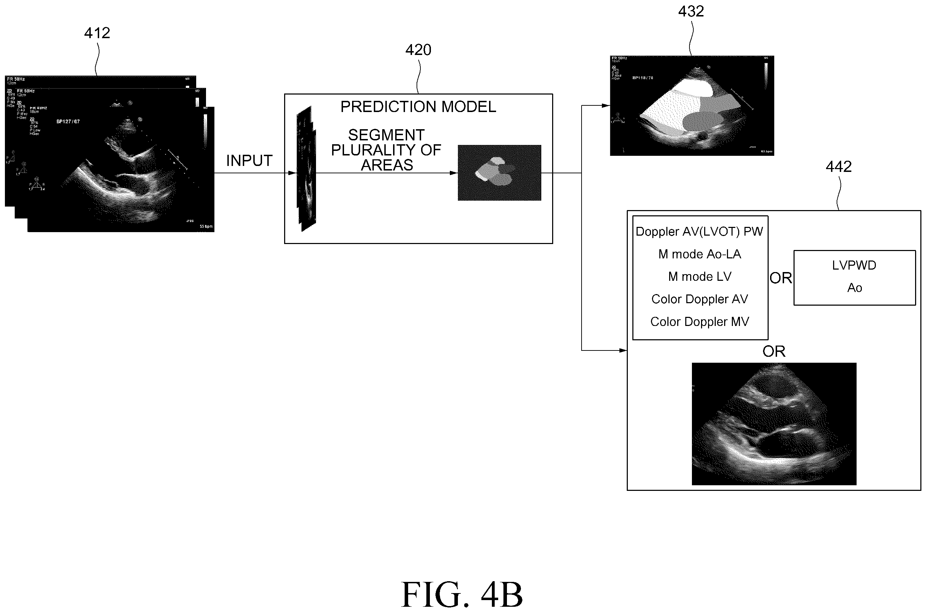

For example, referring to FIG. 4B, in Step S330 in which the measurement parameter is determined, the segmentation result 432 may be output from the prediction model 420 and at the same time, the measurement value-related information 442 including the measurable parameter and/or the measurable mode based on the location of the anatomical structure may be determined and output.

That is, information about the measurable parameter and/or ultrasound image mode and/or segmented anatomical structure and/or measurement value (not illustrated) and/or probe location (not illustrated) may be automatically determined by the prediction model, so that the user may make decisions by determining the measurement value more quickly and quickly.

Meanwhile, the determination of the measurable parameter and/or ultrasound image mode by the aforementioned prediction model may also be performed by a measurement value prediction model that is independent of the prediction model trained to segment the anatomical structure. Here, the measurement value prediction model may be, but is not limited to, a model trained to determine the measurable parameter and/or ultrasound image mode based on the location information of the anatomical structure.

In various embodiments, the prediction model may be a model trained to predict information related to the measurement value during the process of segmenting the area for the anatomical structure.

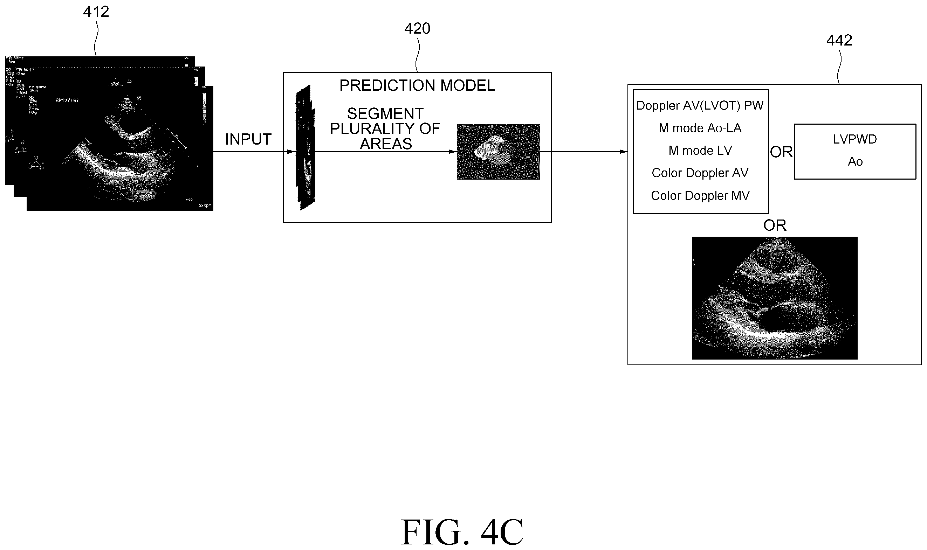

For example, referring to FIG. 4C, in Step S330 where the measurement parameter is determined, the measurement value-related information 442 including the measurable parameter and/or measurable mode may be determined based on the location of the anatomical structure from the prediction model 420. That is, the prediction model 420 may output only the measurement value-related information 442 without outputting the segmentation result and provide the measurement value-related information 442 to the user.

Here, the prediction model 420 may be a model trained to predict the measurable parameter and/or measurable mode or the like based on the positional relationship of the anatomical structure.

This allows the user to quickly obtain only the information related to the determination of the measurement value.

Referring again to FIG. 3, at Step S330 in which the measurement parameter is determined, an evaluation is performed on each of the segmented anatomical structures, and the measurable parameter may be determined based on the evaluation.

Here, for each anatomical structure, the aforementioned location-based connectivity meter, the uncertainty score (for example, entropy meter, mutual information between model posterior probabilities (for example, Bayesian Active Learning by Disagreement; BALD), variation ratio and mean standard deviation), and the corresponding capacity meter in terms of volume may be evaluated.

That is, the evaluation results for each anatomical structure may be provided, making it possible to determine the measurable parameters from low-quality images.

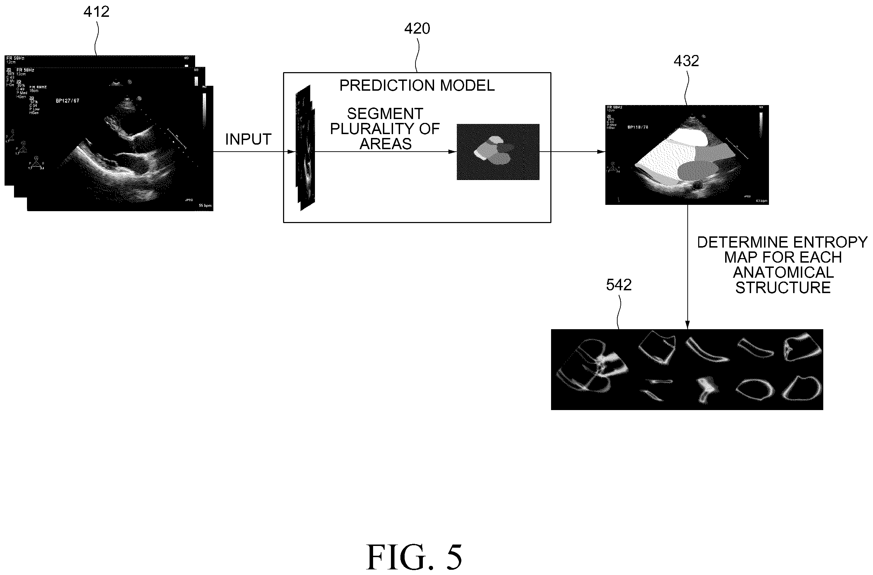

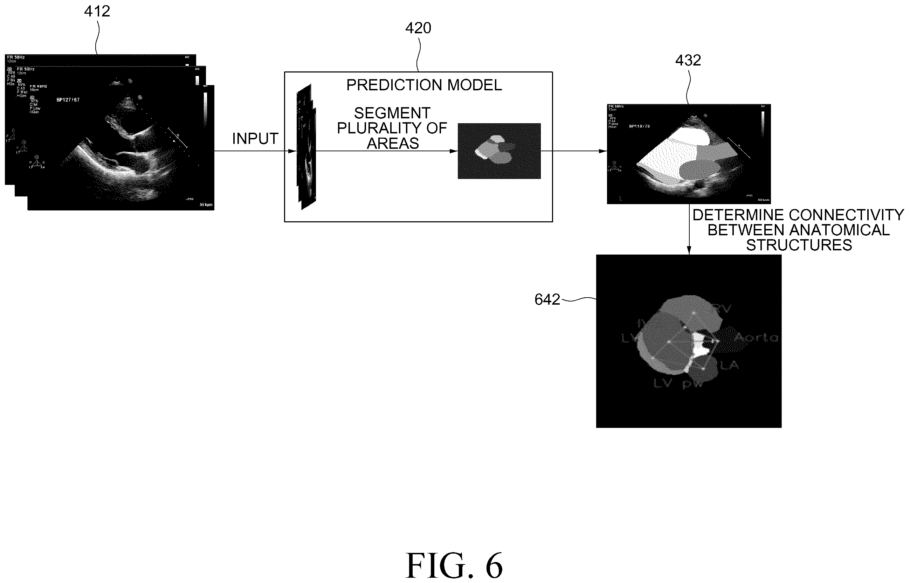

For example, referring to FIGS. 5 and 6 together, an entropy map 542 and a connectivity meter 642 for each anatomical structure may be determined and provided based on the segmentation result 432 for the anatomical structure of the prediction model 420.

In various embodiments, a step of determining guidelines within the ultrasound image based on the segmented anatomical structure may be further performed to enable acquisition of images in different ultrasound modes from the received ultrasound image.

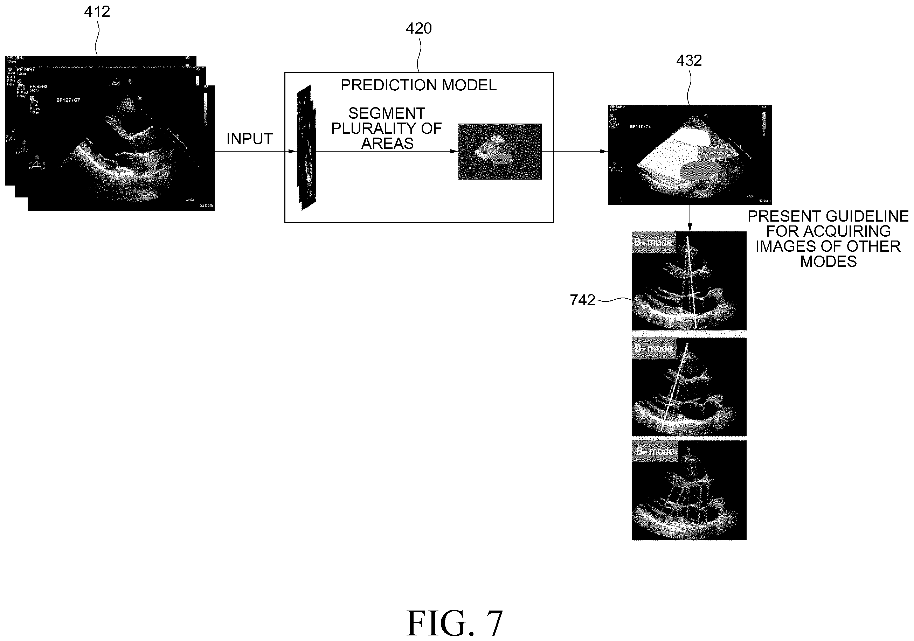

For example, referring to FIG. 7 together, a guideline 742, such as a line for obtaining a Doppler mode or color Doppler mode image, or a box for obtaining an M-mode image, may be determined and displayed in a B-mode image based on the segmentation result 432. Accordingly, the user may more easily obtain images of other modes for determining the measurement value. That is, images of various modes may be obtained and the measurement value may be determined using a handheld type device with a smaller screen than a general ultrasound diagnostic device.

In various embodiments, the user may be prompted for a selection of the ultrasound mode or measurement parameter that are determined to be measurable.

For example, when an input is received for a specific ultrasound mode (view), an image corresponding to the ultrasound mode may be output and provided to the user. Furthermore, when an input is received for a specific parameter, a value corresponding to the measurement value may be output and provided to the user.

That is, in various embodiments of the present disclosure, various information associated with a measurement value may be called and provided according to a user's input.

Accordingly, the medical staff may easily identify the measurement value from the acquired cardiac ultrasound image according to the method for providing the guideline according to various embodiments of the present disclosure, thereby quickly proceeding with the ultrasound analysis step and establishing more accurate decision-making and treatment plans.

Meanwhile, the determination of the prediction and the measurement value for the anatomical structure is not limited to the above-described embodiments.

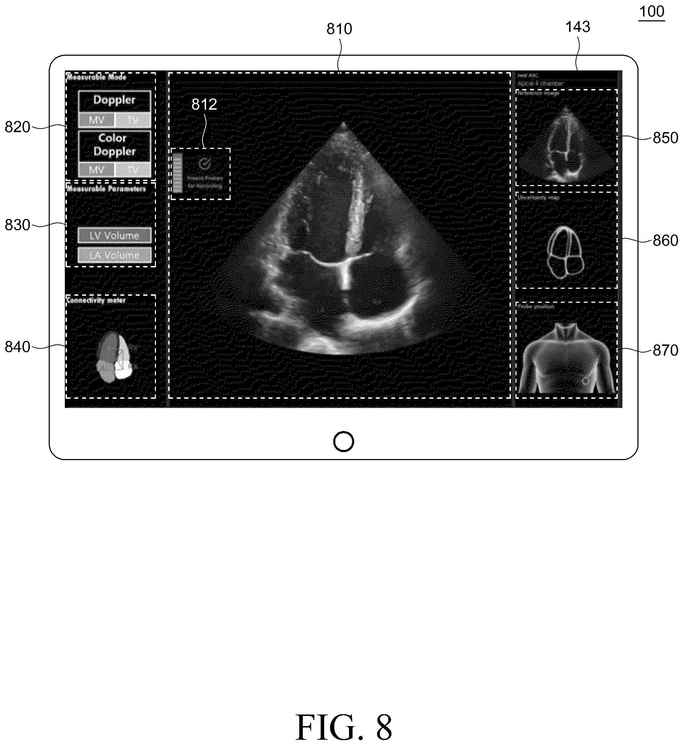

Hereinafter, a user interface according to the method for providing the guideline according to various embodiments of the present disclosure will be exemplarily described with reference to FIG. 8.

Referring to FIG. 8, in various embodiments, the information associated with the measurement value determined from the guideline providing server 300 is provided as the user interface through the touch screen 143 of the medical staff device 100.

More specifically, the user interface includes a still cut image 810 in which names of the anatomical structures are displayed, which are determined to be obtainable for the measurable parameter and/or mode from the images acquired during the diagnosis using the cardiac ultrasound probe. In this case, the still cut image 810 includes an image quality display field 812 that indicates the quality level of the image. The user may check the ultrasound mode obtainable from the still cut image 810 through the measurable mode display field 820. Furthermore, the user may check the measurement value obtainable from the still cut image 810 through the measurable parameter display field 830.

In various embodiments, the user interface includes a connectivity meter display field 840 that provides information about the evaluated connectivity meter for each of the cardiac anatomical structures within the still cut image 810.

The user may check the original image of the still cut image 810 through an original image display field 850.

In various embodiments, the user interface includes an uncertainty score display field 860 that provides information about the uncertainty scores (for example, entropy meter, mutual information between model posterior probabilities (for example, Bayesian Active Learning by Disagreement (BALD), variance ratio, and mean standard deviation) evaluated for each of the cardiac anatomical structures within the still cut image 810.

In various embodiments, the user interface includes a probe position display field 870 that tracks and provides the location of the probe.

According to the method for providing the guideline according to various embodiments of the present disclosure, the user may visually recognize information related to quantification of the cardiac structure more quickly.

### Evaluation 1: Evaluation of Prediction Model

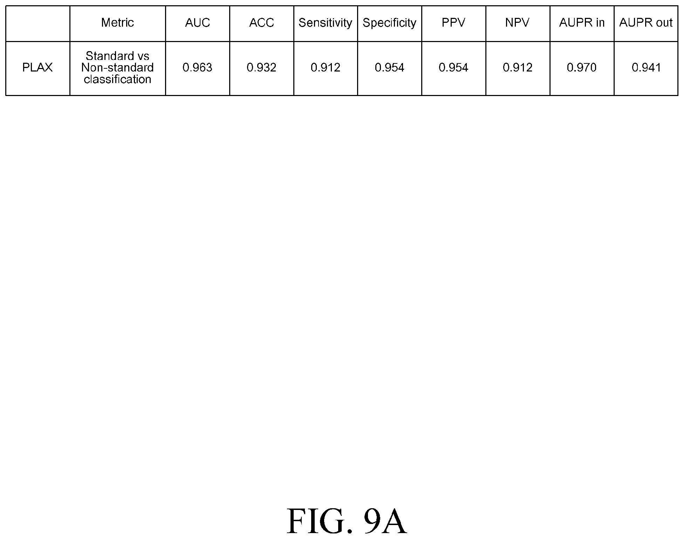

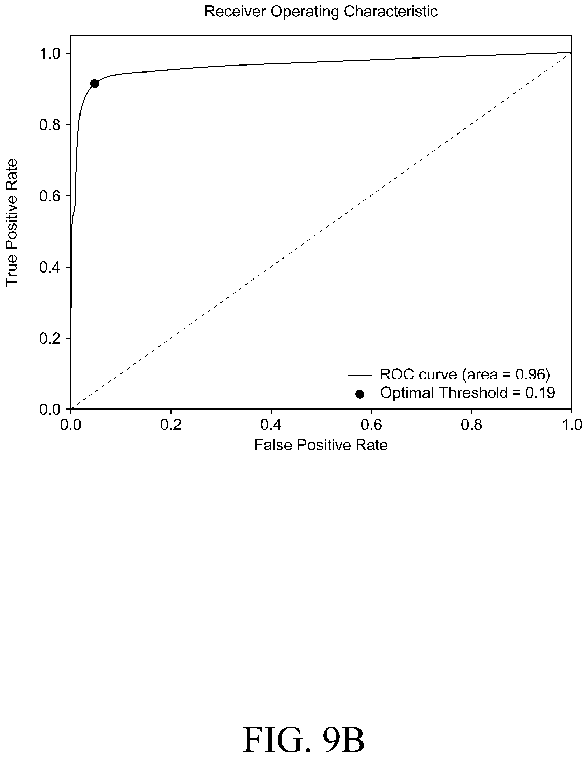

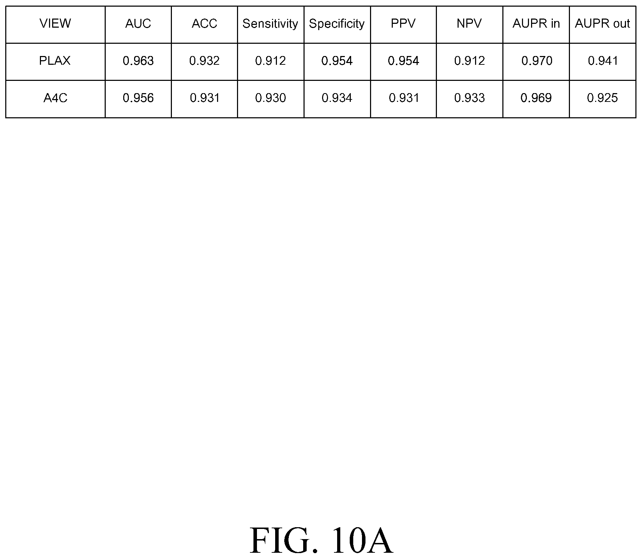

Hereinafter, with reference to FIGS. 9A and 9B and 10A to 10C, the evaluation results of the prediction model applied to various embodiments of the present disclosure will be described. FIGS. 9A and 9B and 10A to 10C illustrate the evaluation results of the prediction model applied to the method for providing the guideline according to various embodiments.

This evaluation was performed based on the PLAX cross-sectional view for the prediction model and/or the standard cross-sectional view and non-standard cross-sectional view classification of the quality meter for A4C.

More specifically, referring to FIGS. 9A and 9B, the prediction model is shown to have excellent diagnostic performance, with a sensitivity of 0.912, a specificity of 0.954, and a particularly high AUC of 0.963 in the PLAX cross-sectional view.

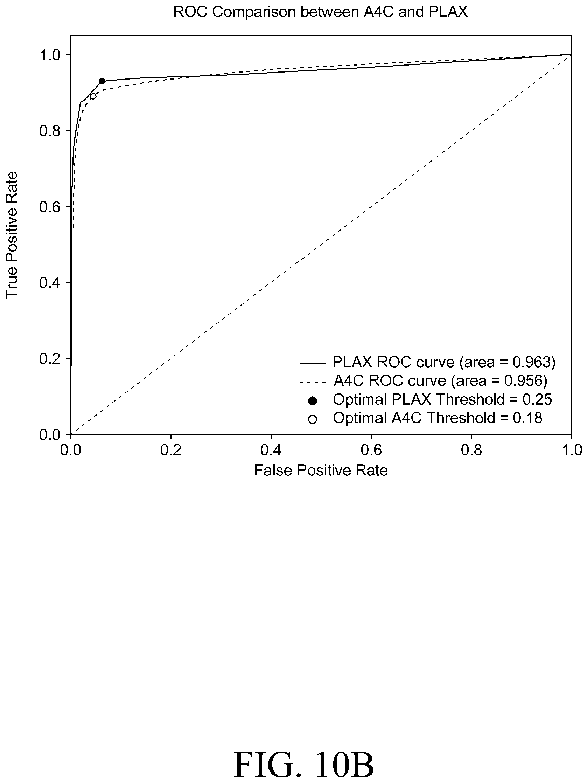

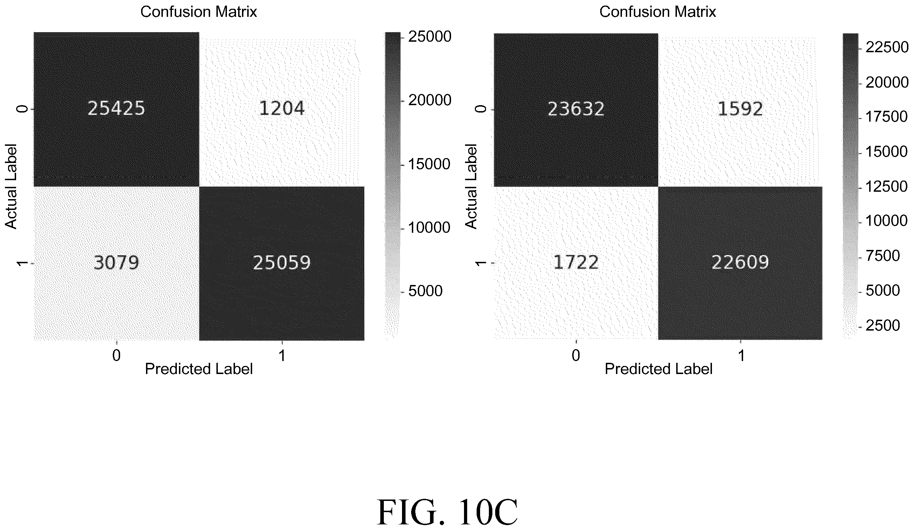

Referring further to FIGS. 10A and 10B, the prediction model shows a high AUC close to 0.96 in both the PLAX cross-sectional view and the A4C cross-sectional view, and referring to the matrix in FIG. 10C, the prediction model appears to show a high AUC of 0.9 or more.

That is, the present disclosure can provide rapid and reliable analysis results for cardiac ultrasound images having various cross-sectional views regardless of the skill level of medical staff, and can contribute to establishing faster and more accurate decision-making and treatment plans at the image analysis stage.

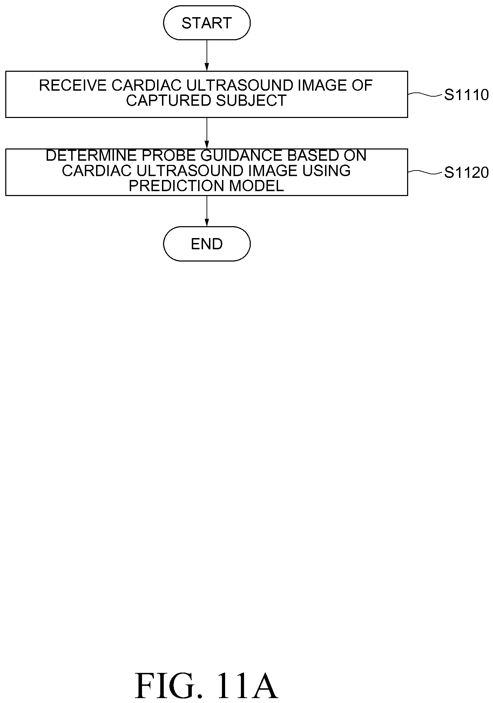

Hereinafter, a method for providing a guideline for a cardiac ultrasound image according to another embodiment of the present disclosure will be specifically described with reference to FIGS. 11A to 11F.

FIGS. 11A to 11F exemplarily illustrate a method for providing a guideline for a cardiac ultrasound image according to one embodiment of the present disclosure.

First, referring to FIG. 11A, a cardiac ultrasound image of a captured subject is received (S1110), and probe guidance is determined based on the cardiac ultrasound image using a prediction model (S1120).

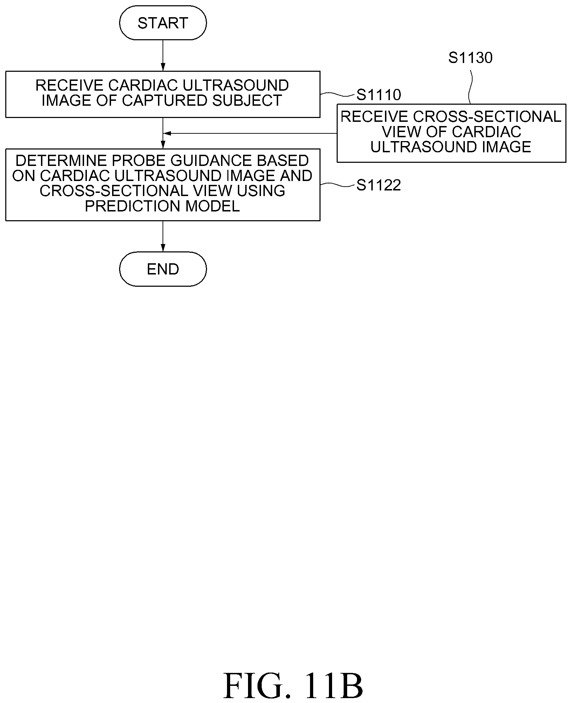

Referring to FIG. 11B, in various embodiments of the present disclosure, the cardiac ultrasound image of the captured subject is received (S1110), the cross-sectional view of the cardiac ultrasound image is received (S1130), and the probe guidance is determined based on the cardiac ultrasound image and the cross-sectional view using the prediction model (S1122).

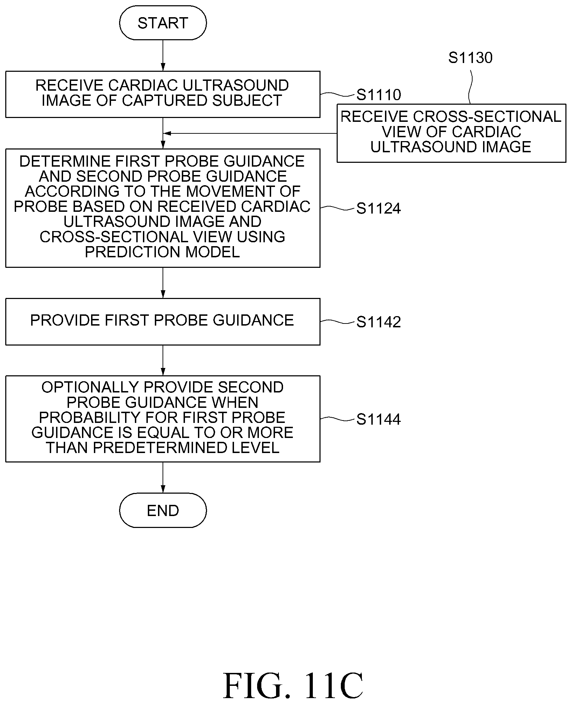

Referring to FIG. 11C, in various embodiments of the present disclosure, the cardiac ultrasound image of the captured subject is received (S1110), a cross-sectional view of the cardiac ultrasound image is received (S1130), and the first probe guidance and the second probe guidance according to the movement of the probe are determined based on the cardiac ultrasound image and the cross-sectional view using the prediction model (S1124).

In this case, the second probe guidance is defined as guidance with greater movement of the probe than the first probe guidance, the first probe guidance includes at least one probe operation guidance of Hold, Probe Head Tilt Down, Probe Head Tilt Up, Probe Head Rock Right, Probe Head Rock Left, Probe Head Tilt Right, Probe Head Tilt Left, Probe Head Rock Down, Probe Head Rock Up, Probe Rotate Clockwise, and Probe Rotate Counter-clockwise, and the second probe guidance includes at least one probe operation guidance of Slide Up, Slide Down, Slide Left, and Slide Right.

In more various embodiments of the present disclosure, the prediction model is configured to probabilistically predict each of the first probe guidance and the second probe guidance.

Next, the first probe guidance is provided (S1142), and when the probability for the first probe guidance is equal to or more than a predetermined level, the second probe guidance is optionally provided (S1144).

That is, the information providing method according to various embodiments of the present disclosure may be configured to provide the probe guidance in stages according to the skill level of the user or the movement of the probe.

More specifically, the prediction model first provides the first probe guidance based on the ultrasound image, and when the result of the user's operation following the guidance satisfies a predetermined level of reliability, the second probe guidance may be optionally provided to guide more precise operation.

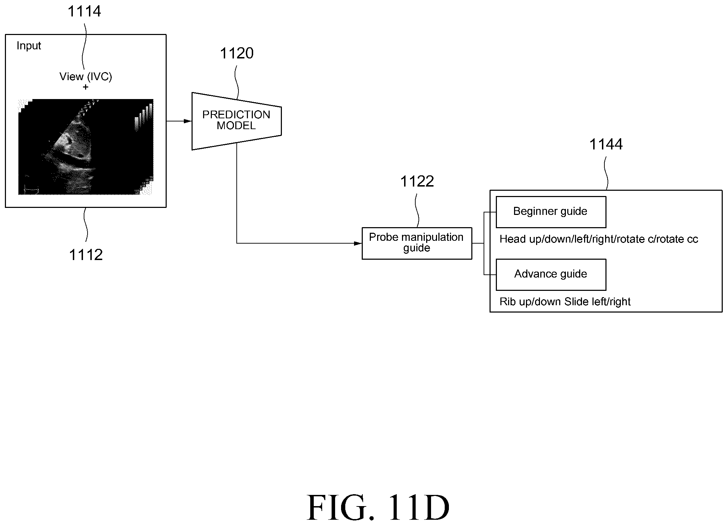

Referring also to FIG. 11D, in various embodiments of the present disclosure, a prediction model 1120 may input a cardiac ultrasound image 1112 and a cross-sectional view 1114 and determine guidance 1122 for the probe operation required by the examiner.

More specifically, the prediction model 1120 may extract features from the cardiac ultrasound image to determine the probe operation required for the input cross-sectional view and output the probe operation guidance 1122.

In this case, two levels of guidance may be provided by considering the size of the probe movement, and the first probe guidance (beginner guidance) having a relatively small probe movement size and the second probe guidance (advanced guidance) 1144 having a relatively large probe movement size may be determined, respectively.

For example, the first probe guidance may provide the guidelines related to small/precise movement and rotation operations of the probe (Head Up/Down, Left/Right, Rotate Clockwise/Counterclockwise) to help the beginners follow along easily. Meanwhile, the second probe guidance may include guidance related to larger operations (Rib Up/Down, Slide Left/Right) to help experienced users obtain more accurate ultrasound images.

Accordingly, the guidance may be received so as to learn basic operations such as Tilt, Rock, and Rotation and find a standard view in a state of fixing the starting point position of the probe. Moreover, when fine correction is still needed even though a standard view is formed, the guidance may be received to obtain an optimal ultrasound image by performing a sliding operation through greater movement.

As a result, the information providing method according to various embodiments of the present invention may contribute to maintaining consistency in ultrasonic imaging and obtaining more accurate images by providing stepwise guidance based on the size of probe movement.

In more various embodiments of the present disclosure, the prediction model may input the cardiac ultrasound image, segment the anatomical structure of the heart, and determine the probe guidance according to the movement of the ultrasound probe based on the segmentation result.

In more various embodiments of the present disclosure, the prediction model may input the cardiac ultrasound image, classify the cross-sectional view, and determine the probe guidance according to the ultrasound probe movement corresponding to the cross-sectional view.

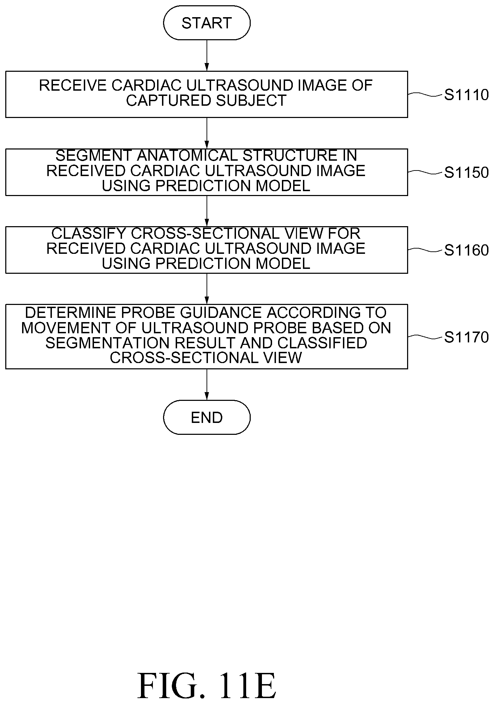

More specifically, with further reference to FIG. 11E, in various embodiments of the present disclosure, the cardiac ultrasound image of the captured subject is received (S1110), and the anatomical structure is segmented in the received cardiac ultrasound image using the prediction model trained to segment the anatomical structure of the heart and classify a cross-sectional view for the input cardiac ultrasound image, by inputting the cardiac ultrasound image (S1150). Then, the cross-sectional view for the received cardiac ultrasound image is classified using the prediction model (S1160), and the probe guidance according to the movement of the ultrasound probe is determined based on the segmentation result and the classified cross-sectional view (S1170).

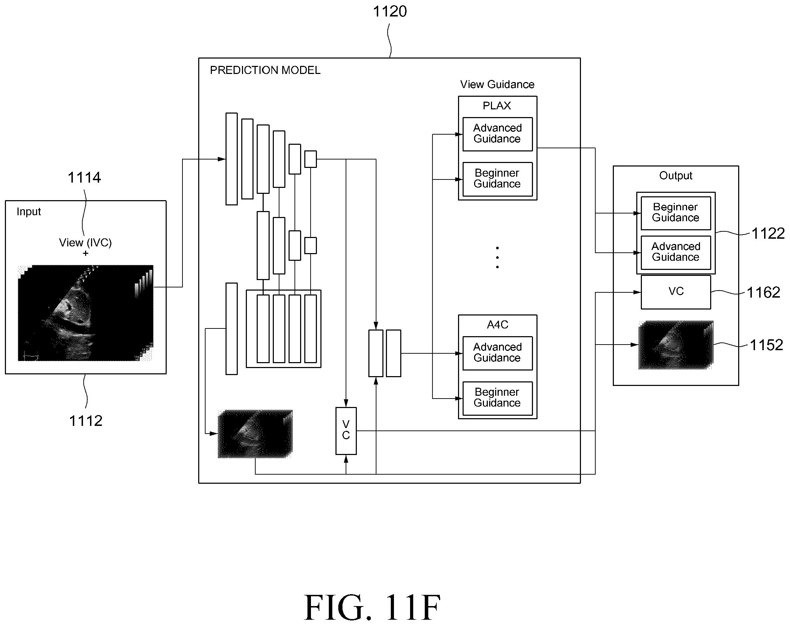

Referring to FIG. 11F together, the cardiac ultrasound image 1112 of the captured subject and the cross-sectional view 1114 of the image are input to the prediction model 1120, and the prediction model may segment the anatomical structures based on the same and then classify the cross-sectional view to output a cross-sectional view classification result (VC) 1162 and a segmented anatomical structure 1152.

In this case, by using a network based on a fully convolutional network (FCN) to segment the anatomical structure of the heart and classify the cross-sectional view of the image by inputting the received cardiac ultrasound image 1112, the prediction model 1120 may segment the anatomical structure of the heart and also optionally calculate the uncertainty score for the segmentation result of the anatomical structure. In addition, the CNN-based network is used to classify the cross-sectional view, and through this, it can be classified whether the image corresponds to a specific cross-sectional view such as PLAX, PSAX, or A4C.

The prediction model 1120 may extract features from the segmented anatomical structure and the cross-sectional view classification result, and perform a merging process to determine probe operation guidance based on the extracted features. In this process, the prediction model 1120 may comprehensively analyze the features of the segmented anatomical structure and determine the operation guidance 1122 of the probe corresponding to the cross-sectional view.

More specifically, the prediction model 1120 may provide the basic probe operation of the first probe guidance (beginner guidance) for beginner examiners, or may provide more precise operations of the second probe guidance (advanced guidance) for experienced users.

The output probe guidance 1122 is provided to the examiner, and the examiner may operate the probe with reference to the probe guidance.

As a result, the prediction model 1120 used in various embodiments of the present disclosure may segment the anatomical structure, classify the cross-sectional view at the same time, extract key features based on the results, comprehensively analyze the features, and then provide optimal probe operation guidance. Through this, the present disclosure may provide a customized guideline according to the probe movement of the user and contribute to improving the quality of cardiac ultrasound image in real time.

Meanwhile, in more various embodiments of the present disclosure, the prediction model may be a model trained to distinguish between a normal cross-sectional view and an odd (abnormal) cardiac ultrasound image by inputting the cardiac ultrasound images.

More specifically, the prediction model may determine whether the captured cardiac ultrasound image contains a medically valid cross-sectional view and detects an abnormal ultrasound image (for example, when an image is captured at the wrong angle, when the ultrasound probe is not placed at a correct position, when the anatomical structure is not clearly visible, or the like).

To this end, the prediction model may set a criterion for maintaining the correct cross-sectional view, and classify an image that does not match the criterion as the odd cardiac ultrasound image.

As a result, the method for providing the guideline according to the embodiment of the present disclosure may analyze the quality of the cardiac ultrasound image in real time by utilizing the prediction model trained to classify the odd cardiac ultrasound image, detect an incorrect cross-sectional view, and provide an optimal probe operation method for correcting the incorrect cross-sectional view. Through this, it supports the examiner to acquire more accurate and stable cardiac ultrasound images, and maintains consistent image quality regardless of the examiner's skill level.

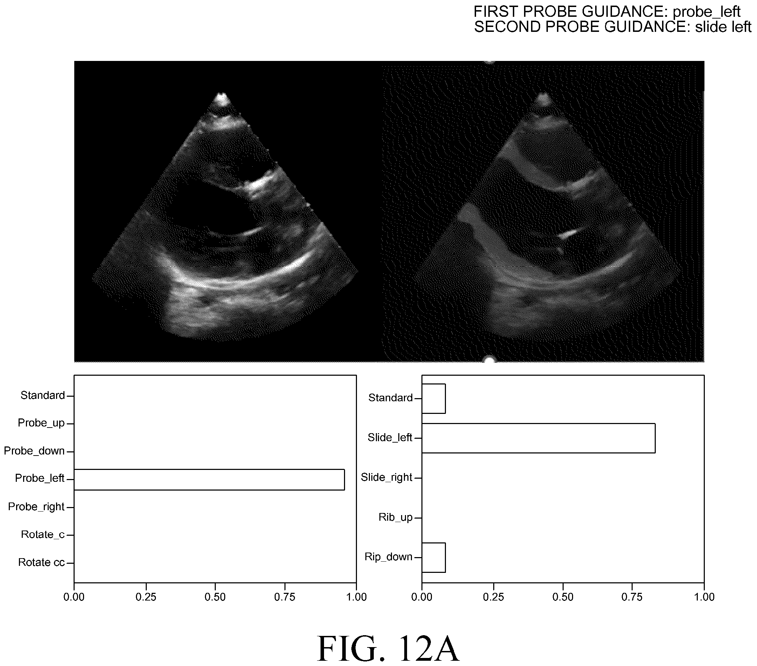

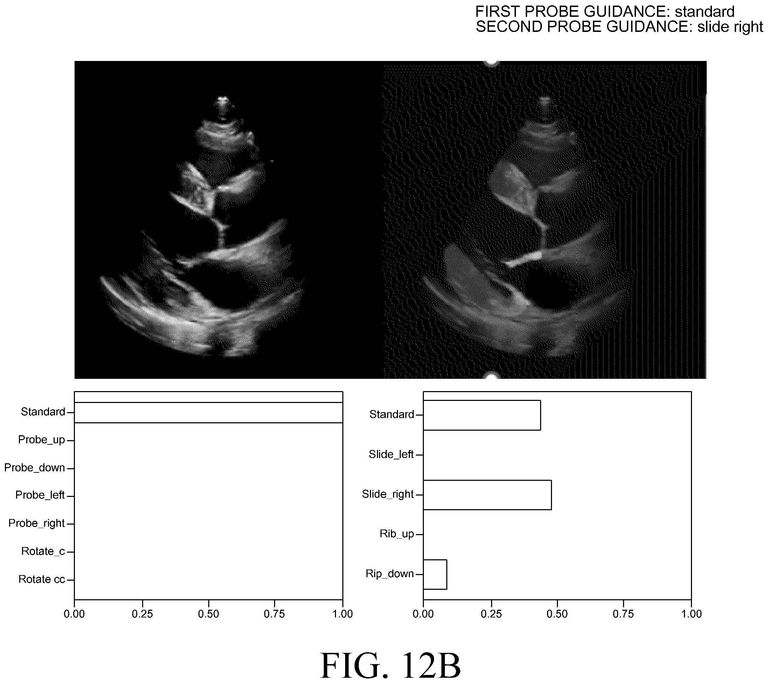

Next, referring to FIGS. 12A and 12B, in various embodiments of the present disclosure, the segmentation result of the cardiac ultrasound image may be output in real time and the probe guidance based on the result may be probabilistically predicted and provided.

Referring to the display field on the left, the actual captured cardiac ultrasound image is displayed, and the image on the right illustrates the results of automatically segmenting the anatomical structure, displayed in color, so that the location and shape of the structure may be intuitively confirmed. This segmentation process may be automatically performed through the prediction model and may be updated in real time.

In addition, referring to the guidance information filed at the upper right, the first probe guidance (for example, probe_left or standard) and the second probe guidance (for example, slide_left or slide_right) for the current probe operation may be probabilistically predicted and provided. In this case, the guidance prediction may be performed based on the segmented structure and the cross-sectional view classification results, and the optimal probe operation direction may be suggested to the examiner in real time.

Referring to the probability graph at the bottom right, the user interface may visually display the predicted probabilities for various probe operation options, providing the examiner with an intuitive idea of how likely a particular operation is to be appropriate.

More specifically, in FIG. 12A, the probabilities of the operations of the first probe guidance of the probe left movement (probe_left) and the second probe guidance operation of sliding to the left (slide_left) are illustrated to be the highest, and in FIG. 12B, the probabilities of the operations of the first probe guidance of a standard (standard) and the second probe guidance operation of sliding to the right (slide_right) are illustrated to be the highest. This may mean that the prediction model according to various embodiments of the present disclosure predicts that the corresponding operation is necessary.

In this case, in the case of the second probe guidance, when the first probe guidance corresponds to the standard, it may be provided optionally, but is not limited thereto.

As a result, in various embodiments of the present disclosure, the results of segmenting the anatomical structure of the cardiac ultrasound image in real time through the user interface and the probabilistically predicted probe operation guidance based on the segmented results may be provided.

That is, the present disclosure may support more precise ultrasound image acquisition by providing intuitive visual feedback through the user interface.

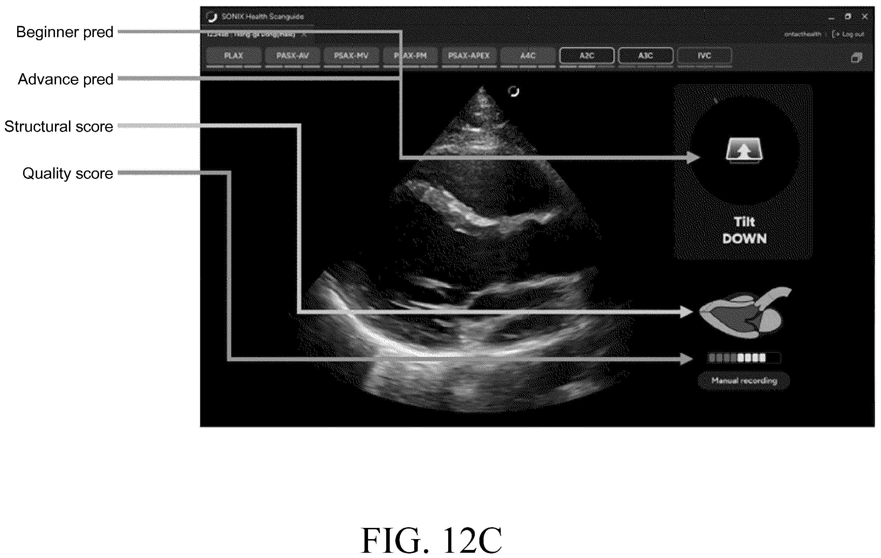

Referring to FIG. 12C together, according to various embodiments of the present disclosure, quality evaluation of the ultrasound image and probe guidance may be predicted in real time through the user interface, and intuitive visual feedback may be provided to the user.

More specifically, the predicted cross-sectional view classification results are displayed at the top, allowing the examiner to check which cardiac cross-sectional view the currently captured ultrasound image corresponds to. In addition, the probe guidance may be predicted according to the probe movement of the examiner, and the beginner guidance (beginner pred) and the advance guidance (advance pred) according to the probe movement may be provided, respectively.

Furthermore, the guidance on the probe operation direction to be adjusted in real time along with the captured ultrasound image is provided through the user interface.

In addition, the quality and structural evaluation scores of the captured image may be provided to the examiner through the user interface. In this case, the structural score and the quality score are output and provided, and the examiner may quantitatively evaluate the accuracy of the anatomical structure recognition and the overall image quality of the currently captured image through the structural score and the quality score.

As a result, the present disclosure may help users obtain more accurate and consistent ultrasound images by analyzing the ultrasound image in real time and providing the probe guidance determined based on predicted cross-sectional view and image quality evaluation results.

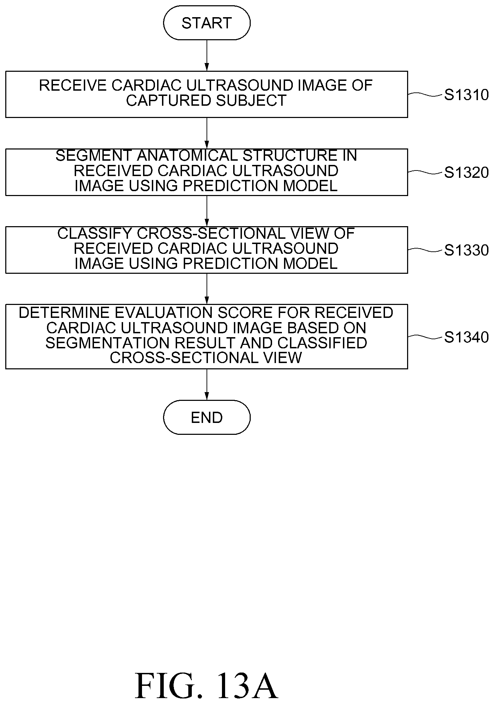

Hereinafter, with reference to FIGS. 13A to 13E, a method for providing a guideline for a cardiac ultrasound image according to another embodiment of the present disclosure will be specifically described.

FIGS. 13A to 13E exemplarily illustrate a method for providing a guideline for a cardiac ultrasound image according to one embodiment of the present disclosure.

First, referring to FIG. 13A, the cardiac ultrasound image of the captured subject is received (S1310), the cardiac ultrasound image is input to segment the anatomical structure of the heart, and the anatomical structure is segmented in the received cardiac ultrasound image using the prediction model trained to classify the cross-sectional view for the input cardiac ultrasound image (S1320). Next, the cross-sectional view of the received cardiac ultrasound image is classified using the prediction model (S1330), and the evaluation score for the received cardiac ultrasound image is determined based on the segmentation result and the classified cross-sectional view (S1340).

In one embodiment of the present disclosure, in Step S1330 of classifying the cross-sectional view, the received cardiac ultrasound image may be classified into at least one cross-sectional view of PLAX, PSAX-AV, PSAX MV, PSAX PM, PSAX APEX, A4C, A3C, and A2C using the prediction model.

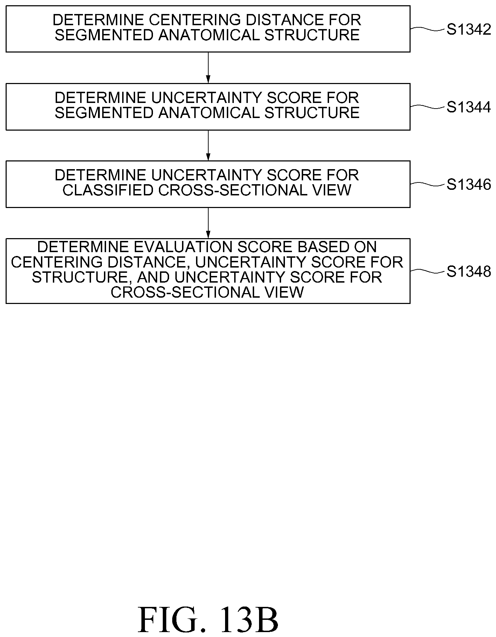

Referring further to FIG. 13B, in various embodiments of the present disclosure, the centering distance for the segmented anatomical structure is determined (S1342), the uncertainty score for the segmented anatomical structure is determined (S1344), and the uncertainty score for the classified cross-sectional view is determined (S1346). Then, the evaluation score may be determined based on the centering distance, the uncertainty score for the structure, and the uncertainty score for the cross-sectional view (S1348).

In more various embodiments of the present disclosure, a first uncertainty score for the overall anatomical structure segmented from the prediction model and a second uncertainty score for each of the anatomical structures segmented from the prediction model may be determined to determine the uncertainty score for the structure.

In more various embodiments of the present disclosure, in order to determine the evaluation score for the cardiac ultrasound image, the structural score defined as the score for evaluating capture of the anatomical structure for the received cardiac ultrasound image may be determined based on the segmentation result for the prediction model and the classified cross-sectional view, and the image quality score defined as the score for evaluating the image quality for the cardiac ultrasound image may be determined.

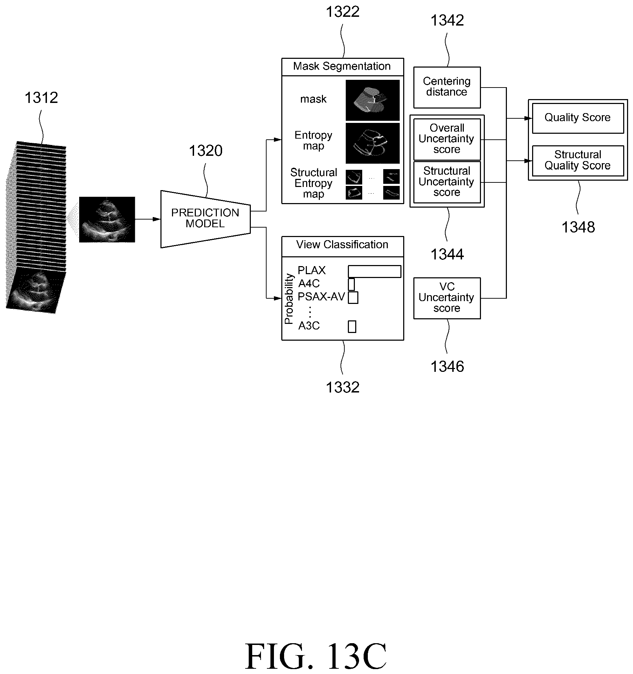

For example, referring to FIG. 13C together, when a cardiac ultrasound image 1312 is input to a prediction model 1320, an anatomical structure 1322 is segmented and a cross-sectional view 1332 is classified and output. Then, a centering distance 1342 for the segmented anatomical structure may be determined, and an uncertainty score 1344 for the overall image and each individual structure may be determined, respectively. In this case, an uncertainty score (VC uncertainty score) 1346 for the classified cross-sectional view may also be determined.

Then, based on the determined centering distance, the uncertainty score for the structure, and the uncertainty score for the cross-sectional view, the evaluation score is calculated (S1348), and at this time, the structural score and the image quality score 1348 may be determined, respectively. These scores may be used as a criterion for evaluating whether the cardiac ultrasound image shot by the user is correctly captured and for quantitatively analyzing the quality of the image.

As a result, the method for providing the guideline according to various embodiments of the present disclosure may automate the analysis and quality evaluation of the anatomical structure of the cardiac ultrasound image, and may support maintaining consistent image quality regardless of the skill level of the examiner.

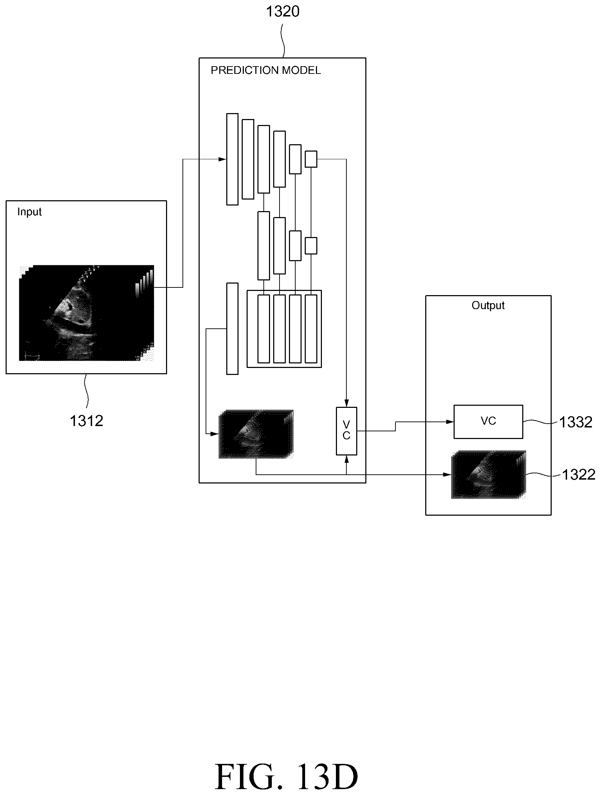

Referring further to FIG. 13D, the prediction model 1320 may receive the cardiac ultrasound image 1312 as input, extract features through a neural network including R2dp1d and FPN structures, analyze the anatomical structure, and classify the cross-sectional view.

According to an aspect of the present disclosure, the prediction model 1320 may perform the cross-sectional view classification and the segmentation of the anatomical structure for all cross-sectional cardiac ultrasound images of PLAX, PSAX-AV, PSAX MV, PSAX PM, PSAX APEX, A4C, A3C and A2C.

According to an aspect of the present disclosure, the prediction model 1320 may input a cardiac ultrasound image, which is a video image including a plurality of frames.

In this case, the R2dp1d module may be designed to simultaneously train spatial and temporal features in the cardiac ultrasound image by combining 2D convolutions, which enables temporal feature analysis that reflects not only individual frames but also structural changes over time.

Additionally, the feature pyramid network (FPN) module may be configured to extract features at multiple resolutions to segment various cardiac anatomical structures more precisely.

Once this feature extraction process is completed, the anatomical structure may be detected using the segmentation network based on a fully convolutional network (FCN), and the cross-sectional view may be classified, so that the cross-sectional view classification result 1332 and the segmented image 1322 may be output.

That is, the prediction model 1320 used in various embodiments of the present disclosure may more precisely analyze the anatomical structure of the heart by considering not only a single ultrasound frame but also consecutive frames, classify the cross-sectional view at the same time, and generate highly reliable information for the quality evaluation of the ultrasound image and provision of guidance.

Meanwhile, the prediction model 1320 may be based on the R2dp1d module and FPN as described above, but is not limited thereto, and may perform the anatomical structure analysis and cross-sectional view classification of the cardiac ultrasound image by applying various deep learning architectures.

For example, the prediction model 1320 may be capable of extracting features that consider long-distance dependencies by applying a transformer-based network, and may also reflect features between consecutive ultrasound frames by utilizing a model that trains time-series information, such as 3D CNN or ConvLSTM. In addition, by applying the model that includes a self-attention layer, predictions that emphasize important structural features and reduce noise may be performed.

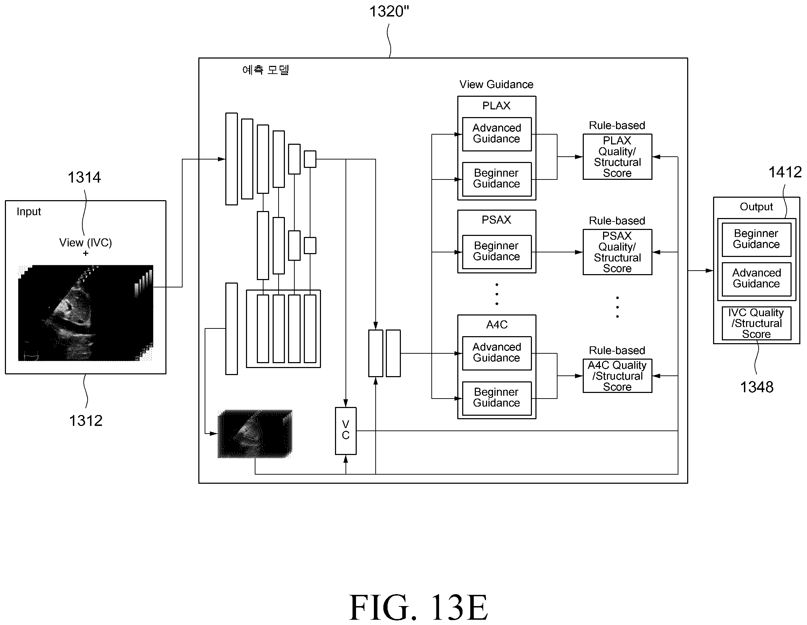

Referring to FIG. 13E, in more various embodiments of the present disclosure, the cardiac ultrasound image 1312 of the captured subject and the cross-sectional view 1314 of the image are input to a prediction model 1320", and the prediction model may segment the anatomical structure based on these and then classify the cross-sectional view to output the cross-sectional view classification result (VC) 1332.

**In** this case, in various embodiments of the present disclosure, quality and structural evaluation scores may be calculated within the prediction model in a rule-based manner.

More specifically, the prediction model 1320" may internally calculate quality and structural evaluation scores for each cross-sectional view (for example, PLAX, PSAX, and A4C), and this process may be performed in a rule-based manner. To this end, the prediction model 1320" may extract and analyze features based on the segmented anatomical structures and cross-sectional view classification results, and then quantify the features to calculate quality and structural evaluation scores. The scores calculated in this way may be provided as the evaluation scores for individual cross-sectional views, such as PLAX quality/structural score and PSAX quality/structural score.

Therefore, the prediction model applied to various embodiments of the present disclosure may be designed so that an evaluation score may be directly calculated within the model, through which the model may automatically determine the image quality and provide real-time feedback to the user. As a result, the examiner may be provided with probe operation guidance based on the immediate quality evaluation results, thereby obtaining more accurate cardiac ultrasound images.

Meanwhile, in more various embodiments of the present disclosure, the prediction model may be the model trained to distinguish between the normal cross-section view and the odd (abnormal) cardiac ultrasound image by inputting the cardiac ultrasound image.

More specifically, the prediction model may determine whether the captured cardiac ultrasound image contains a medically valid cross-sectional view and detect an abnormal ultrasound image (for example, when image is captured at the wrong angle, when the ultrasound probe is not placed at a correct position, when the anatomical structure is not clearly visible, or the like).

To this end, the prediction model may set a criterion for maintaining the correct cross-sectional view, and classify an image that does not match the criterion as the odd cardiac ultrasound image.

As a result, the method for providing the guideline according to the embodiment of the present disclosure may analyze the quality of the cardiac ultrasound image in real time by utilizing the prediction model trained to classify the odd cardiac ultrasound image, detect an incorrect cross-sectional view, and provide an optimal probe operation method for correcting the incorrect cross-sectional view. Through this, it supports the examiner to acquire more accurate and stable cardiac ultrasound images, and maintains consistent image quality regardless of the examiner's skill level.

### Evaluation 2: Evaluation of Prediction Model

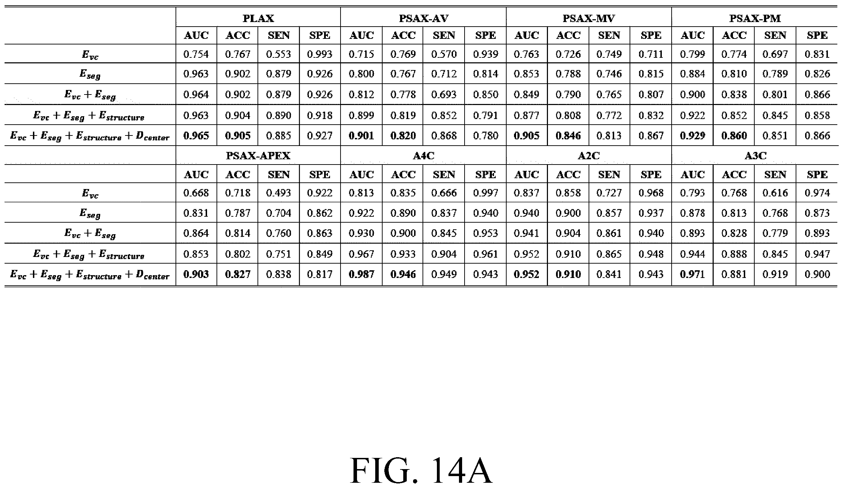

Hereinafter, with reference to FIGS. 14A to 14D, the evaluation results of the prediction model applied to various embodiments of the present disclosure are described.

First, referring to FIG. 14A, the results of analyzing the quality evaluation index using the entropy value to evaluate the performance of the prediction model according to various embodiments of the present disclosure are illustrated. In this case, the prediction performance was evaluated based on Area Under the Curve (AUC), Accuracy (ACC), Sensitivity (SEN), and Specificity (SPE). Furthermore, the performance of the prediction model was compared by the cross-sectional view (PLAX, PSAX-AV, PSAX-MV, PSAX-PM, PSAX-APEX, A4C, A2C, and A3C).

More specifically, when the prediction model with only a module performing the cross-sectional view classification was used, the AUC and ACC were shown to be low overall (see E_{vc}). At this time, when the module performing the anatomical structure classification was added, the prediction performance was shown to improve (see E_{vc} + E_{seg}), and in particular, when the module for calculating the image quality score was added together, the highest prediction performance was shown for all cross-sectional views (E_{vc} + E_{seg} + E_{structure} + E_{center}).

The above results may imply that the prediction model of the present disclosure may precisely evaluate the quality of ultrasound images by utilizing various features, and that quality evaluation is possible with high accuracy.

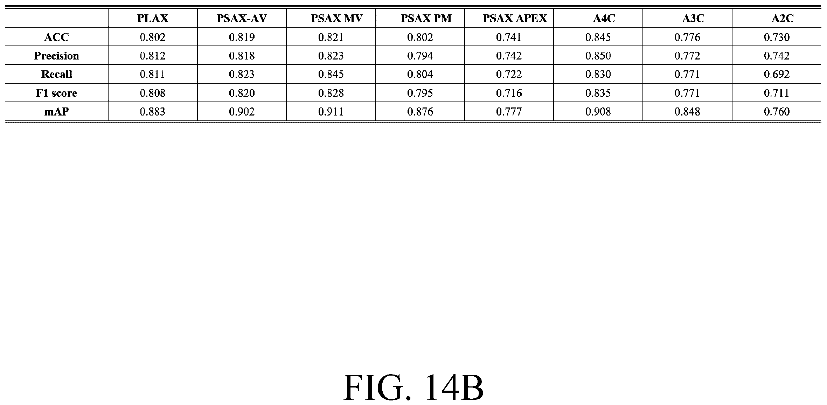

Next, referring to FIG. 14B, the results of measuring the probe movement prediction performance of the prediction model for each cross-sectional view (PLAX, PSAX-AV, PSAX-MV, PSAX-PM, PSAX-APEX, A4C, A3C, and A2C) are illustrated.

More specifically, in terms of accuracy, the prediction model was shown to exhibit relatively high prediction performance in PSAX-MV and A4C cross-sectional views.

For the F1 Score index, the highest value of 0.835 was observed in the A4C cross-sectional view, and an F1 Score value of 0.828 was observed in the PSAX-MV cross-sectional view. This may mean that the prediction model showed excellent probe movement prediction performance in the corresponding cross-sectional view.

Furthermore, referring to the results of the mean accuracy (mAP) analysis, the PSAX-MV cross-sectional view showed the highest performance at 0.911 and the A4C cross-sectional view at 0.908, which may mean that the prediction model operates with high reliability for predicting probe movement in the corresponding cross-sectional view.

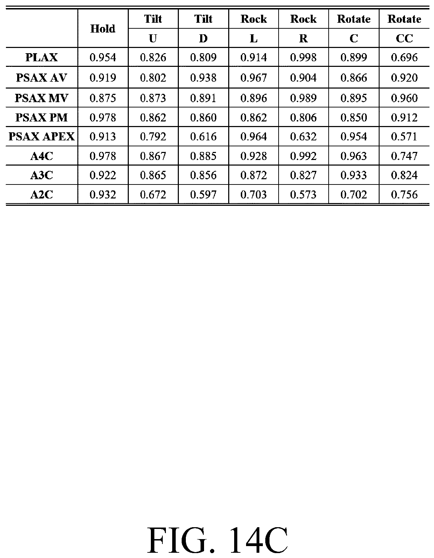

Next, referring to FIG. 14C, the prediction performance for a specific probe movement is measured for each cross-sectional view, and the results of analyzing the prediction accuracy of each movement are illustrated.

More specifically, the rock right movement in the PLAX cross-sectional view showed a high prediction accuracy of 0.998, and the rock right movement in the PSAX-AV and PSAX-MV cross-sectional views showed high prediction performances of 0.967 and 0.989.

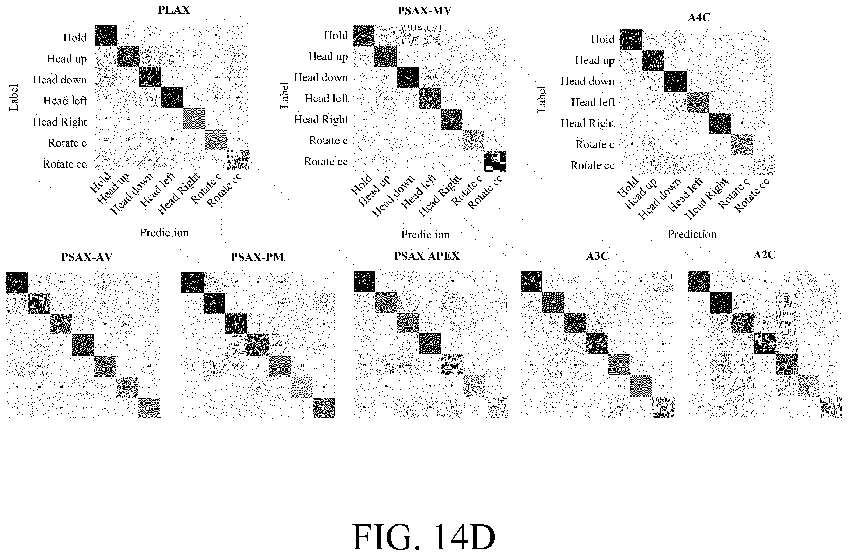

Referring further to FIG. 14D, a matrix is illustrated to analyze the probe movement prediction performance of the prediction model in more detail.

In this case, when analyzing the relationship between the predicted probe operation direction and the actual operation direction for each cross-sectional view (PLAX, PSAX-MV, A4C, PSAX-AV, PSAX-PM, PSAX-APEX, A3C, and A2C), the more values located on the diagonal, the more accurately the model predicted.

More specifically, the prediction model was shown to predict the probe movement with high accuracy in the cross-sectional views of PLAX, PSAX-MV, and A4C.

The above results may suggest that the prediction model according to various embodiments of the present disclosure may accurately predict the probe movement for each cross-sectional view, and that guidance optimization considering characteristics by cross-sectional view is possible to provide accurate probe operation.

That is, the present disclosure provides a prediction model that automatically segments the anatomical structures of the heart and classifies the cross-sectional views, thereby enabling rapid and accurate recognition of specific anatomical structures in the ultrasound images. Through this, more standardized cardiac ultrasound images may be obtained without relying on the experience of the examiner.

Furthermore, the present disclosure automatically calculates the evaluation score based on the image segmentation result and cross-sectional view classification, thereby enabling quantitative evaluation of the quality of the captured ultrasound image in real time.

**In** this way, the examiner may receive evaluation scores and determine in real time whether the image quality meets certain standards, and if the image quality is insufficient, the probe guidance may be provided.

That is, the present disclosure can provide highly reliable analysis results for the cardiac ultrasound images regardless of the skill of the examiner, and can contribute to establishing more accurate decision-making and treatment plans at the image analysis stage.

Although the embodiments of the present disclosure have been described in more detail with reference to the attached drawings, the present disclosure is not necessarily limited to these embodiments, and various modifications may be made without departing from the technical idea of the present disclosure. Accordingly, the embodiments disclosed in the present disclosure are not intended to limit the technical idea of the present disclosure, but to explain it, and the scope of the technical idea of the present disclosure is not limited by these embodiments. Therefore, it should be understood that the embodiments described above are exemplary in all aspects and not restrictive. The protection scope of the present disclosure should be interpreted by the following claims, and all technical ideas within a scope equivalent thereto should be interpreted as being included in the scope of the rights of the present disclosure.

## Claims

1. A method for providing a guideline for a cardiac ultrasound image implemented by a processor, the method comprising:
receiving the cardiac ultrasound image of a captured subject; and
determining probe guidance based on the received cardiac ultrasound image by using a prediction model trained to determine the probe guidance according to movement of an ultrasound probe by inputting the cardiac ultrasound image.

2. The method according to claim 1, further comprising receiving a cross-sectional view of the cardiac ultrasound image,
wherein the determining the probe guidance further includes determining the probe guidance based on the received cardiac ultrasound image and the received cross-sectional view using the prediction model.

3. The method according to claim 1, wherein the determining the probe guidance includes determining first probe guidance and second probe guidance according to the movement of the probe based on the received cardiac ultrasound image using the prediction model.

4. The method according to claim 3, wherein the second probe guidance is defined as guidance with greater movement of the probe than the first probe guidance,
the first probe guidance includes at least one probe operation guidance of Hold, Probe Head Tilt Down, Probe Head Tilt Up, Probe Head Rock Right, Probe Head Rock Left, Probe Head Tilt Right, Probe Head Tilt Left, Probe Head Rock Down, Probe Head Rock Up, Probe Rotate Clockwise, and Probe Rotate Counter-clockwise, and
the second probe guidance includes at least one probe operation guidance of Slide Up, Slide Down, Slide Left, and Slide Right.

5. The method according to claim 4, wherein the prediction model is configured to probabilistically predict each of the first probe guidance and the second probe guidance, and
the method further includes
providing the first probe guidance, and
selectively providing the second probe guidance when probability for the first probe guidance is equal to or greater than a predetermined level.

6. The method according to claim 1, wherein the prediction model is further configured to segment an anatomical structure of a heart by inputting the cardiac ultrasound image, and
the determining the probe guidance further includes
segmenting the anatomical structure in the received cardiac ultrasound image using the prediction model, and
determining the probe guidance based on the segmentation result using the prediction model.

7. The method according to claim 1, wherein the prediction model is further configured to classify a cross-sectional view of the input cardiac ultrasound image by inputting the cardiac ultrasound image, and
the determining the probe guidance further includes
classifying the cross-sectional view of the received cardiac ultrasound image using the prediction model, and
determining the probe guidance corresponding to the classified cross-sectional view using the prediction model.

8. The method according to claim 7, wherein the classifying the cross-sectional view includes classifying the received cardiac ultrasound image into at least one cross-sectional view of PLAX, PSAX-AV, PSAX MV, PSAX PM, PSAX APEX, A4C, A3C, and A2C using the prediction model, and
the determining the corresponding probe guidance includes determining the probe guidance for the at least one cross-sectional view.

9. A method for providing a guideline for a cardiac ultrasound image implemented by a processor, the method comprising:
receiving the cardiac ultrasound image of a captured subject;
segmenting an anatomical structure of a heart by inputting the cardiac ultrasound image and using a prediction model trained to classify a cross-sectional view of the input cardiac ultrasound image, thereby segmenting the anatomical structure in the received cardiac ultrasound image;
classifying the cross-sectional view of the received cardiac ultrasound image using the prediction model; and
determining an evaluation score for the received cardiac ultrasound image based on the segmentation result and the classified cross-sectional view.

10. The method of claim 9, wherein the determining the evaluation score for the cardiac ultrasound image includes
determining a centering distance for the segmented anatomical structure,
determining an uncertainty score for the segmented anatomical structure,
determining an uncertainty score for the classified cross-sectional view, and
determining the evaluation score based on the centering distance, the uncertainty score for the structure, and the uncertainty score for the cross-sectional view.

11. The method of claim 10, wherein the determining the uncertainty score for the structure includes
determining a first uncertainty score for all of the segmented anatomical structures, and
determining a second uncertainty score for each of the segmented anatomical structures.

12. The method of claim 9, wherein the determining the evaluation score for the cardiac ultrasound image includes
determining a structural score defined as a score for evaluating capture of the anatomical structure for the received cardiac ultrasound image based on the segmentation result and the classified cross-sectional view, and
determining an image quality score defined as a score for evaluating an image quality for the cardiac ultrasound image.

13. The method of claim 9, wherein the prediction model is a model trained to classify an odd cardiac ultrasound image by inputting the cardiac ultrasound images.

14. The method of claim 9, wherein the cardiac ultrasound image is a video including a plurality of frames.
